# EUROPEAN PATENT SPECIFICATION

(11) **EP 4 146 810 B1**
(45) Date of publication and mention of the grant of the patent: **28.01.2026**
(21) Application number: 21725435.8
(22) Date of filing: 04.05.2021
(51) Int. Cl.: C12N 15/867, C12N 5/10, C12M 3/00

(54) **LENTIVIRAL VECTOR MANUFACTURING PROCESS IN PACKED BED BIOREACTOR**
VERFAHREN ZUR HERSTELLUNG EINES LENTIVIRALEN VEKTORS IN EINEM FESTBETTBIOREAKTOR
PROCÉDÉ DE FABRICATION DE VECTEUR LENTIVIRAL DANS UN BIORÉACTEUR À LIT TASSÉ

(30) Priority: 04.05.2020 EP 20172806
(43) Date of publication of application: 15.03.2023
(73) Proprietor: AGC Biologics S.p.A., 20091 Bresso (MI) (IT)
(72) Inventor: NERI, Margherita, 20091 Bresso (MI) (IT); COTA, Manuela, 20091 Bresso (MI) (IT); ALLIEVI, Luca, 20091 Bresso (MI) (IT); TESTASECCA, Angela, 20091 Bresso (MI) (IT); BONFANTI, Francesca, 20091 Bresso (MI) (IT); VALLANTI, Giuliana, 20091 Bresso (MI) (IT)
(74) Representative: Studio Torta S.p.A.
(86) International application number: PCT/EP2021/061673
(87) International publication number: WO 2021/224227

(56) References cited:
- WO-A1-2018/007873
- WO-A1-2020/037249
- HANNA M. LEINONEN ET AL: "Preclinical Proof-of-Concept, Analytical Development, and Commercial Scale Production of Lentiviral Vector in Adherent Cells", MOLECULAR THERAPY - METHODS & CLINICAL DEVELOP, vol. 15, 1 December 2019 (2019-12-01), GB, pages 63 - 71, XP055738788, ISSN: 2329-0501, DOI: 10.1016/j.omtm.2019.08.006
- HANNA M. LEINONEN ET AL: "Benchmarking of Scale-X Bioreactor System in Lentiviral and Adenoviral Vector Production", HUMAN GENE THERAPY, vol. 31, no. 5-6, 1 March 2020 (2020-03-01), GB, pages 376 - 384, XP055738793, ISSN: 1043-0342, DOI: 10.1089/hum.2019.247
- ALEXANDRA MCCARRON ET AL: "Transient Lentiviral Vector Production Using a Packed-Bed Bioreactor System", HUMAN GENE THERAPY METHODS, vol. 30, no. 3, 1 June 2019 (2019-06-01), pages 93 - 101, XP055738805, ISSN: 1946-6536, DOI: 10.1089/hgtb.2019.038
- A J VALKAMA ET AL: "Optimization of lentiviral vector production for scale-up in fixed-bed bioreactor", GENE THERAPY, vol. 25, no. 1, 5 October 2017 (2017-10-05), GB, pages 39 - 46, XP055738781, ISSN: 0969-7128, DOI: 10.1038/gt.2017.91

## Description

### FIELD OF THE INVENTION

The present invention relates to the field of large scale manufacturing of lentiviral vectors by multi-plasmid DNA transient transfection of packaging cells performed in packed bed bioreactor systems.

### BACKGROUND TO THE INVENTION

Lentiviral vectors or lentivirus (LVVs) are a gene delivery vehicle largely applied in gene therapy applications. LVVs are known to offer many advantages including the ability to i) transduce dividing and non-dividing cells, ii) permanently integrate into the host cell genome thus providing sustained gene expression in the host cells and any progeny (Naldini et. al. 2006). Moreover, LVVs are considered safer due to their low immunogenicity and toxicity, as well as their low rate of insertional mutagenesis and tumorigenesis in comparison with onco-retroviral vectors. LVVs can be pseudotyped with envelope proteins deriving from other viruses thus conferring a broad tropism or specificity for particular target cells (Chronin et al 2005). For all these reasons, LVVs are largely used in different gene therapy applications such as for example, the treatment of several genetic disease including metachromatic leukodystrophy (Biffi et al. 2013) Wiskott-Adrich Syndrome (Aiuti et al 2013) bthalassemia (Cavazzana-Calvo et al. 2010) or X-Linked adrenoleukodystrophy (Cartier et al 2009). All these clinical applications have the final aim to correct genetic deficiencies by delivering a gene of interest into CD34+ Hematopoietic stem cells to be infused into the patient. Further examples of clinical applications in which LVVs are used as gene delivery vehicles is the adoptive cellular therapy using T cells genetically engineered to express Chimeric Antigen Receptors (CAR) or T-Cell Receptors. Chimeric Antigen Receptors (CARs) are recombinant receptors that recognize a specific protein or antigen expressed on a target cell. Once expressed in T lymphocytes, which is then called a CAR-T cell, or other cells of the immune system, CARs are able to redirect a specific immune response against all cells that express the antigen they bind to. The most largely explored clinical application of CARs is the cancer immunotherapy, which consists in the infusion of cells of the immune system, such as T cells or NK cells, carrying a CAR targeted to a tumour antigen. Such cells are able to generate a strong antitumour response against cells expressing the antigen targeted by the CAR (Sadelain et al., Cancer Discovery. 2013. 3(4):388-98).

Several CAR-T cell candidates are now in clinical development and different successful cases are emerging. The Food and Drug Administration (FDA) recently approved tisagenlecleucel (Kymriah^{®}), the first CAR T-cell therapy indicated for the treatment of patients up to 25 years of age affected by B-cell precursor acute lymphoblastic leukaemia (ALL) that is refractory or in second or later relapse.

Growing interest in the use of LVVs has created a strong demand for large volumes of vectors for preclinical, toxicology and clinical studies as well as ultimately for manufacturing of drug products approved for the market.

The most largely used methods for manufacturing of the LVVs require the multi-plasmid transient trasfection of packaging cells (such as 293 cell line and its derivatives 293 T, 293 E or 293Vec) in monolayer culture using T-flasks or multi-tray system such as cell factories. Different processes have been disclosed which employ different transfection reagents including calcium phosphate (Segura et al. 2007).

The present invention is focused on the development of a process for the manufacturing of LVVs in large scale comprising transient multi plasmid transfection of cells performed in a packed bed bioreactor. The inventors found the conditions to perform the manufacturing process characterized by high productivity and quality of the LVVs, as well as reproducibility at small and at large scale, and an effective reduction of the costs taking into consideration the total duration of the process and the consumption of the most critical materials.

The prior art discloses LVVs manufacturing processes based on transient transfection of packaging cells performed in packed bed bioreactor but there is no indication of the essential technical features or the performance of the process according to the present invention.

For example, in Valkama et al. Optimization of lentiviral vector purification for scale-up in fix bed bioreactor Gene Therapy (2018) 25, 39-46; the authors disclose a process for the manufacturing of LVVs, performed by transient transfection of 293T cells in the packed bed bioreactor system iCellis Nano. The authors report the performance of 6 runs in iCellis Nano high compaction, having a surface area of 4 m² and 4 runs in iCellis Nano low compaction having a surface area of 2.67 m². The DNA quantity to be used in transfection is reported to be one of the critical parameters for the yield of the process. The authors recommend to use DNA in a total amount in the range 300 to 400 ng/cm², with PEI as transfection reagent, and with a ratio DNA:PEI equal to 1:1. It is clearly stated that transfection efficiency is reduced to only 5% by using 100ng/cm² of DNA. The entire run, except the 4 or 6 hours of transfection, is performed applying perfusion of fresh medium into the bioreactor i.e. during cell culture, after transfection, and harvesting. Harvest in perfusion of recombinant LVVs particles starts 24 hours after transfection, and it continues for the following 48 hours (i.e.: transfection and harvesting steps require a total of 4 days). It is reported that the definition of the harvesting window between 24 to 72 hours from transfection derives from the observation that LVVs productivity at 6 hours from transfection is low. Perfusion is performed at fix constant rate or at variable rate, in certain runs a complete change of medium is performed by perfusion 1 or 2 days after transfection.

WO 2018/ 007873 discloses a process substantially similar to that described in Valkama et al. 2018, including the optimized DNA quantity in transfection of 300ng/cm² to 400ng/cm², and the observation that the transfection efficiency is reduced to only 5% by using 100ng/cm² of DNA. WO 2018/ 007873 also discloses that, in order to achieve an optimal DNA concentration in the transfection mix (i.e.: the mix obtained by combining DNA and PEI) a large volume of transfection mix is required which would require a full exchange of medium in the bioreactor. The inventors state that at large scale, the full exchange of medium (i.e.: the draining of almost the total amount of cell culture medium present in the bioreactor) is not a practical process step, because it requires time and may be influencing on cell viability due to the fact that during draining, stirring is closed and the cells in the upper carriers are without medium. To overcome this limitation, the transfection mix is added to the bioreactor by recirculation.

In Mc Carron et al. Transient Lentiviral Vector Production Using a Packed Bed Bioreactor System (2019) Human Gene Therapy 30 (3) 93-101, the authors disclose a method for the manufacturing of LVVs in a single use packed bed bioreactor Bio-BLU (Eppendorf) having a surface area of 18m², and a working volume of 3L (i.e.: still a limited amount of medium to be managed for empting and filling the bioreactor that does not require long time). LVVs manufacturing is performed by transient transfection of 293T cells using 4,79mg of total DNA with PEI transfection reagent (ratio DNA:PEI 1:3). The entire run of production is performed in batch mode (including cell culture, cell transfection and harvesting). After transfection the cell culture medium is removed from the system, and 3L of fresh medium is added to the bioreactor. Harvesting of the cell culture medium containing recombinant LVV particles is performed by collecting medium at 48 and 72 hours post transfection (i.e.: transfection and harvesting steps require a total of 4 days). Sampling performed at 24, 48 and 72 hours post transfection shows that, in this process, the highest productivity is obtained at 72 hours post transfection, the authors suggest to evaluate productivity also after 72 hours post transfection.

In Leinonen et al. Preclinical Proof of Concept, Analytical Development and Commercial Scale production of lentiviral vector in adherent cells (2019) Molecular Therapy 15, 63-71, the authors disclose a method for the manufacturing of LVVs by transient transfection of 293T cells in iCellis Nano Bioreactor having a surface area of 2,67m² or at larger scale using iCellis500 Bioreactor having a surface of 100m² or 333m². Transfection is performed using DNA quantity at 300ng/cm² or 200ng/m² and PEI transfection reagent. The transfection mix is added to the bioreactor and incubated for 6 hours in absence of perfusion. All runs are performed using perfusion or recirculation mode in order to supply the fresh medium to the cells in all phases of the process except the transfection. Perfusion (or recirculation) is applied during cell culture, after 6 hours of transfection and during collection of LVV. LVVs collection is started 24 hours after transfection up to 72 hours post transfection. Before starting LVV collection, i.e.: 24 hours after transfection, a complete change of medium is performed by perfusion using the same medium of perfusion post transfection until complete medium change. This step is reported to reduce productivity but, together with the addition into the bioreactor of endonuclease already during the production phase, it results to have positive effect on the purity of the final product in terms of DNA contaminants in the final bulk containing LVVs. When applied at large scale, in a packed bed bioreactor having a surface area of 333m², the process results to have a good physical titer even higher than as expected, but an infective viral titer less than as expected. Particularly, the total viral transfection units (TU) obtained with the process applied in the bioreactor having surface area of 333m² appears to be similar to that obtained in a bioreactor having surface area of 100 m² (3,3 fold smaller bed size) using a process based on transfection with higher total DNA quantity and without post transfection medium change. Moreover, the authors report that a single endonuclease treatment performed at the end of the run is not sufficient to provide DNA clearance. For this reason, the authors introduce in the process a first endonuclease treatment during production phase in the bioreactor and a second treatment after harvest in order to reduce DNA contaminants. Other methods for manufacturing LVVs are disclosed in Leinonen et al. Human Gene Therapy, vol. 31, no. 5 and 6, p. 376 (2020), WO2020037249, Valkama et al., Gene therapy, vol. 25, no. 1, page 39 (2017) and WO2018007873.

There is a need to develop a reproducible manufacturing methods that allows obtaining LVVs in high quantity and quality to satisfy the growing demand of this kind of gene delivery vehicles for production of genetically modified cell therapy products. The present invention addresses this need.

### SUMMARY OF THE INVENTION

The present invention relates to a method for the manufacturing of LVV vectors in a packed bed bioreactor according to claim 1. In particular, the inventors focused on those parameters and technical conditions to perform the process that resulted to cause an improvement of the productivity (physical titre, infectious viral titre and infectivity), the quality of the final product (low DNA and host cells proteins contaminants) and a lower consumption of critical reagents (such as the DNA used in transfection) in respect to the amount and functionality of the final product. Moreover, the inventors found that by applying the claimed process, it was possible to reduce up to a total of three days the period necessary to perform transfection and collection of LVVs, which was disclosed to be 4 days in the processes disclosed in prior art. Particularly, while the prior art discloses processes operated totally in batch mode or in perfusion mode, the present invention discloses and claims a process in which both technical modes are applied and combined to obtain an effective process.

According to the present invention, there is provided a method for the manufacturing of lentiviral vectors in a packed bed bioreactor according to claim 1. In particular, it is provided a method for the manufacturing of lentiviral vectors in a packed bed bioreactor by multi-plasmid DNA transient transfection comprising the following steps:
(i) Inoculating the cells
(ii) Culturing the cells for expansion for at least one day
(iii) Transfecting the cells by adding into the bioreactor a transfection mix containing DNA transfecting particles obtained by mixing multi-plasmid DNA and PEI and incubating the cells with the DNA transfecting particles in absence of perfusion or recirculation of fresh medium
(iv) changing the medium in batch mode by draining from the bioreactor the cell culture medium containing the DNA transfecting particles and adding fresh medium to the cell culture
(v) Starting, immediately after the addition of the fresh medium under point iv, the collection of the recombinant lentiviral vectors by harvesting, in perfusion mode, the cell culture medium from the bioreactor.

Moreover, according to a further aspect of the invention, The inventors identified the ideal range of total DNA to be used for multi-plasmid transient transfection in a process for the manufacturing of LVVs performed in a packed bed bioreactor. The use of the DNA in such range allows to obtain LVVs having good quality and a productivity comparable or even higher than that observed with processes disclose in prior art which make use of greater amounts of total DNA in transfection, with a subsequent important reduction of costs.

Therefore, according to the present invention, there is provided a method for the manufacturing of lentiviral vector in a packed bed bioreactor comprising transiently transfecting the cells using multi-plasmid DNA in a total amount between 45ng/cm² and 100ng/cm² of the surface area of the packed bed and the PEI transfection reagent.

### EMBODIMENTS

According to the present invention, in a first aspect there is provided a method for the manufacturing of lentiviral vectors in a packed bed bioreactor by multi-plasmid DNA transient transfection comprising the following steps:
(i) Inoculating the cells
(ii) Culturing the cells for expansion for at least one day
(iii) transfecting the cells by adding into the bioreactor a transfection mix containing DNA transfecting particles obtained by mixing multi-plasmid DNA and PEI, and incubating the cells with the DNA transfecting particles in absence of perfusion or recirculation of fresh medium
(iv) changing the medium in batch mode by draining from the bioreactor the cell culture medium containing the DNA transfecting particles and adding fresh medium to the cell culture
(v) starting, immediately after the addition of the fresh medium under point iv, the collection of the recombinant lentiviral vectors by harvesting in perfusion mode the cell culture medium from the bioreactor

In one embodiment the steps of transfection (iii), post transfection medium change (iv) and harvesting in perfusion (v) are performed in three days.

In another embodiment the method further comprises a total change of medium between step ii and step iii.

In a further embodiment the cells, under step ii, are cultivated for expansion in perfusion mode. Preferably the cultivation in perfusion mode is performed by supplying in the bioreactor up to the end of the expansion phase the fresh medium in a total amount of at least 0,1 ml/cm² of the surface area of the packed bed.

In a preferred embodiment the cells, under step ii, are cultivated for expansion in recirculation mode. More preferably the cultivation in recirculation mode is performed using as reservoir the fresh medium in a total amount of at least 0,1 ml/cm² of the surface area of the packed bed.

In a preferred configuration of the method of the invention the cells under step iii are incubated with the DNA transfecting particles for a period up to 18 hours, more preferably for a period between 6 and 18 hours, in a more preferred embodiment for a period of 8 hours.

Preferably, in one embodiment the cells under step iii are transfected using the multi-plasmid DNA in a total amount between 45ng/cm² and 100ng/cm² of the surface area of the packed bed. The transfection mix under step iii is prepared using a ratio between DNA amount and PEI amount corresponding to 1:1.

In a preferred configuration of the invention the collection of the recombinant lentiviral vectors under step v is performed by applying perfusion for at least 39 hours.

In another configuration of the invention the collection of the recombinant lentiviral vectors under step v is performed by applying perfusion for 48 hours.

In one embodiment the collection of the recombinant lentiviral vectors under step v is performed by applying perfusion using the same medium used under step v.

In another embodiment the collection of the recombinant lentiviral vectors under step v is performed by applying perfusion using a cell culture medium comprising Fetal Bovine Serum (FBS) in an amount between 2,5% to 10% FBS.

In a further embodiment the collection of the recombinant lentiviral vectors under step v is performed by applying perfusion using a cell culture medium without FBS.

In another aspect of the invention, there is provided a method for the manufacturing of lentiviral vectors in a packed bed bioreactor comprising transiently transfecting the cells using multi-plasmid DNA in a total amount between 45ng/cm² and 100ng/cm² of the surface area of the packed bed and the PEI transfection reagent.

Preferably the invention provides a method for manufacturing of lentiviral vectors in a packed bed bioreactor by multi-plasmid DNA transient transfection comprising the following steps:
a) Inoculating the cells
b) Culturing the cells for expansion for at least one day
c) Transfecting the cells using the multi-plasmid DNA in a total amount between 45ng/cm² and 100ng/cm² of the surface area of the packed bed, and the PEI transfection reagent
d) Harvesting the cell culture supernatant containing the recombinant lentiviral vectors

The transfection is performed using a ratio between DNA amount and PEI amount
corresponding to 1:1.

In another embodiment the method further comprises a total change of medium between the culturing for expansion under step b and the transfection under step c.

In a further embodiment the cells are cultivated for expansion under step b above in perfusion mode. Preferably, the cultivation in perfusion mode is performed by supplying in the bioreactor, up to the end of the expansion phase, the fresh medium in a total amount of at least 0,1 ml/cm² of the surface area of the packed bed.

In a preferred embodiment the cells are cultivated for expansion under step b above in recirculation mode. Preferably, the cultivation in recirculation mode is performed using as reservoir the fresh medium in a total amount of at least 0,1 ml/cm² of the surface area of the packed bed.

In a preferred configuration of the method of the invention, the cells are transfected, under step c above, for a period up to 18 hours, more preferably for a period between 6 and 18 hours. In a most preferred configuration the cells are transfected for 8 hours.

In one embodiment the method further comprises a change of medium between transfection under step c and harvesting under step d above, such change of medium may be performed in perfusion or in batch mode, more preferably in batch mode.

In another embodiment, the harvesting under step d above is performed in batch mode or in perfusion mode.

In one configuration of the invention there is provided a method in which harvesting under step d above is performed in batch mode by collecting the cell culture medium from the bioreactor at 24 and 48 hours from the post transfection change of medium.

In another configuration of the invention there is provided a method in which harvesting under step d above is performed in perfusion mode, applied for at least 39 hours after the post transfection change of medium.

In another configuration there is provided a method in which harvesting under step d above is performed in perfusion mode applied for 48 hours after the post transfection change of medium. In a more preferred configuration of the invention there is provided a method in which harvesting under step d is performed in perfusion mode and the steps from transfection (step c) to harvesting (step d) are performed in three days.

### DETAILED DESCRIPTION OF THE INVENTION

Various preferred features and embodiments of the present invention will now be described by way of non-limiting exemplifying embodiments.

The present invention provides a method for the manufacturing of lentiviral vectors (LVVs) in a packed bed bioreactor by multi-plasmid DNA transient transfection. The method of the invention is focused particularly on the production of LVVs at high quantity, good quality, in large scale and using automated device. The inventors found the optimal conditions to obtain with high reproducibility a bulk production of LVVs having high physical and infectious viral titre and optimal impurity profile.

According to the present invention, there is provided a method the for manufacturing of lentiviral vectors in a packed bed bioreactor by multi-plasmid DNA transient transfection comprising the following steps:
(i) Inoculating the cells
(ii) Culturing the cells for expansion for at least one day
(iii) Transfecting the cells by adding into the bioreactor a transfection mix containing DNA transfecting particles obtained by mixing multi-plasmid DNA and PEI, and incubating the cells with the DNA transfecting particles in absence of perfusion or recirculation of fresh medium
(iv) changing the medium in batch mode by draining from the bioreactor the cell culture medium containing the DNA transfecting particles and adding fresh medium to the cell culture
(vi) Starting, immediately after the addition of the fresh medium under point iv, the collection of the recombinant lentiviral vectors by harvesting in perfusion mode the cell culture medium from the bioreactor.

### Lentiviral vectors (LVVs)

The present invention relates to the manufacturing of LVVs. LVVs are gene delivery vehicles derived from lentiviruses. A detailed list of lentiviruses may be found in Coffin et al ("Retroviruses" 1997 Cold Spring Harbour Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human acquired-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

In a preferred embodiment, the lentiviral vector is derived from an HIV lentivirus, more preferably the lentiviral vector is derived from an HIV-1 lentivirus.

An exemplary lentiviral vector to be used in the method of the invention comprises at least the following portion of lentiviral genome: a) a 5' long terminal repeat (LTR); b) the packaging sequence psi; c) a Rev Response Element (RRE); d) a promoter operably linked to a gene of interest; e) a 3' long terminal repeat (LTR). In a preferred embodiment the U3 region of the 5' LTR is replaced with a heterologous promoter selected from the group consisting of: a cytomegalovirus (CMV) promoter, a Rous Sarcoma Virus (RSV) promoter, or a Simian Virus 40 (SV40); thus rendering lentiviral transcription *tat* independent. In a further preferred embodiment the 3' LTR sequence contains a deletion of the U3 region (i.e. the vector is a self inactivating vector or SIN vector). The lentiviral vector may further comprise a lentiviral central polypurine tract (cPPT) and Woodchuck Hepatitis Virus (WHP) Posttranscriptional Regulatory Element (WPRE)).

### Packed bed Bioreactor

The process according to the present invention makes use of a packed bed bioreactor for production of LVVs. A packed bed bioreactor is a device consisting of a vessel containing or filled or packed with carrier material used as support for the immobilization of cells. Carriers are composed of organic and inorganic material on which the cells are entrapped, thus allowing cell growing in adhesion up to a very high density. The culture medium conditioned with appropriate quantity of O2 and required pH condition, is circulated through the fixed bed to provide nutrients and O2 and to permit cell growth. Different packed bed bioreactors are available working essentially in two possible configurations: 1) the medium is circulated from a conditioning vessel to the fixed bed and back to the conditioning vessel (e.g.: CellCube system Corning); or 2) the fixed bed is integrated in a bioreactor and the medium conditioned within this bioreactor is circulated through this bed (e.g.: iCellis Pall Corporation). In a preferred embodiment the process according to the present invention can be performed in the iCELLis^{®} Single-Use Fixed-Bed Bioreactor Systems as shown in the examples. The iCELLis bioreactor system is an automated, single-use, fixed-bed bioreactor for adherent cell growth in a controlled environment. The bioreactor is available in two format i.e. the iCELLis Nano used for development studies with different possible surface areas for cell growth (units from 0.53 m² to 4 m² of cultivation area) and the iCellis 500 for large scale production having cultivation areas units from 66 to 500 m². Cells grow in polyester macro carriers contained in the fixed bed while the cell culture medium flows through the fixed-bed from the bottom to the top. At the top, the medium falls as a thin film down the outer wall where it takes up O2 back to the reservoir. As used herein the terms "cultivation area" or "surface area" mean the total surface of the packed bed of the bioreactor on which the cells are attached and cultivated.

### Multi-plasmid transient transfection

According to the present invention LVVs are produced in the packed bed bioreactor by multi-plasmid DNA transient transfection of cells i.e. in the method of the invention at least two expression plasmids carrying two different genes are transiently co-transfected into a cell line (the so called packaging cell line). According to a preferred aspect of the invention, the cells are co-transfected at least with: a packaging plasmid carrying lentiviral Gag/Pol genes, a plasmid carrying the gene encoding the envelope protein of interest and the transfer plasmid carrying the essential lentiviral genome elements, as disclose above, and the gene of interest. In another preferred embodiment the lentiviral regulatory protein Rev may be further expressed in *trans* on a fourth separate plasmid. In a further aspect of the invention lentiviral protein tat may be further expressed on a fifth separate plasmid. Examples of suitable *env* genes include, but are not limited to VSV-G env, MLV 4070 env, RD114 env, RD114-TR, RD114pro, baculovirus 5 GP64 env, GALV or envelope proteins derived from Measle virus.

In a preferred embodiment, in the method of the invention the multi-plasmid DNA transfection is performed using a third generation LVVs viral vector system composed by four plasmids i.e.: one plasmid carrying the lentiviral gag/pol genes, one plasmid carrying lentiviral rev gene, one plasmid carrying the gene encoding the envelope protein, preferably the gene encoding the VSV-G envelope protein, and one plasmid carrying the transfer vector including the required portion of the lentiviral genome and the foreign gene of interest.

### Inoculum of the cells into the bioreactor

The manufacturing method according to the present invention comprises the inoculum of the cells i.e.: the introduction of the cells into the bioreactor.

Cells can be inoculated into the bioreactor at a quantity between 1000 cells/cm² and 10000 cells/cm² of the cultivation area of the bioreactor. In another embodiment cells can be cultivated at quantity between 2000 cells/cm² and 8000cells/cm², preferably between 4000 cells/cm² and 6000cells/cm², in a more preferred embodiment 5000 cells/cm² are inoculated into the bioreactor. In order to obtain the appropriate quantity, the cells, following thawing, may be first expanded in cell culture out of the bioreactor. In one embodiment the cells are thawed, expanded up to the appropriate quantity through cell culture in adhesion, and then detached from the support and inoculated into the bioreactor in the appropriate quantity.

Prior art discloses the possibility to use suspension adapted cell lines into a packed bed bioreactor thanks to the use of factors promoting cell adhesion during cell culture in the bioreactor (WO 2016/048556). Therefore, in another embodiment according to the present invention, a suspension adapted cell line is thawed and expanded up to the appropriate quantity through the performance of a cell culture in suspension, and then inoculated into the bioreactor in the presence of a factor that promotes cell adhesion.

All cell lines known to act as packaging cell line for the manufacturing of viral vectors can be used in the present invention. In one embodiment the packaging cell line to be used in the present invention is selected from HEK293, HEK293 T, HEK 293E, HEK 293FT, 293 Vec, TE671, HT1080 or HeLa cell line. In a preferred embodiment the packaging cell line is HEK 293 T.

### Cell expansion

According to the method of the invention, further to the inoculum the cells are cultivated for expansion into the bioreactor for at least one day.

The skilled man may use, in this step, any of the cell cultivation operating conditions known in the art and applicable to the packed bed bioreactor in use, for supplying the cell culture medium containing nutrients to the cells. For example, the iCELLis^{®} Single-Use Fixed-Bed Bioreactor Systems allows to operate cell culturing in three different modes: 1) "in batch mode" according to which the cells are cultivated using a constant volume of cell culture medium into the bioreactor for the required period of time up to the media consumption (i.e.: the exhaustion of the nutrients required for cell growth and the accumulation of cell metabolites) and, upon reaching medium consumption, it is necessary to drain completely the exhausted medium from the bioreactor and fill it with the fresh medium 2) "in perfusion mode" according to which during the cell culture, the fresh medium is added into the bioreactor at a constant flow rate and the exhausted medium is removed at the same flow rate to be wasted or harvested, while maintaining a constant total volume of cell culture medium into the bioreactor and allowing a constant supply of nutrients to the cell culture and a constant removal of cell metabolites; 3) "re-circulation mode" according to which the cells are cultivated in a constant amount of cell culture medium into the bioreactor, which is connected with an external container serving as reservoir of medium, from which additional culture medium is added at a constant flow rate into the bioreactor (as in perfusion mode) and removed at the same flow rate to be carried back to the reservoir. The culture in re-circulation mode allows an increase of the total volume of medium used for the cell culture. Each of the above mentioned operational modes or a combination thereof may be employed in the method of the invention to culture the cells for expansion.

In one embodiment there is provided a method according to the present invention, wherein the cells, are cultivated for expansion for at least 1 day by operating the bioreactor in batch mode. More preferably, when cell culture for expansion is performed in batch mode, at the end of expansion phase, and before transduction, a complete change of medium is performed by draining the total volume of the exhausted cell culture medium from the bioreactor that is then filled with the fresh medium. Alternatively, the post culture change of medium may be performed in perfusion mode, by supplying at fix rate the fresh medium in an amount corresponding to the double volume of the cell culture medium present in the bioreactor. In another embodiment when cell culture for expansion is performed in batch mode, at the end of the expansion phase, and before transduction, the post culture change of medium may be performed in perfusion mode, by supplying at fix rate fresh medium, in a total amount corresponding to the same volume of the cell culture medium present in the bioreactor.

In another embodiment there is provided a method according to the present invention wherein the cells, are cultivated for expansion for at least 1 day by operating the bioreactor in perfusion mode. In a preferred configuration of the method of the invention the cell culture in perfusion mode is performed by supplying up to the end of the expansion phase the fresh medium in a total amount of at least 0,1 ml/cm² of the surface area of the packed bed.

In a preferred embodiment there is provided a method according to the present invention wherein the cells, are cultivated for expansion for at least 1 day by operating the bioreactor in recirculation mode. In a preferred configuration of the method of the invention, the cell culture in recirculation mode is performed using as reservoir, the additional medium in a total amount of at least 0,1 ml/cm² of the packed bed.

The goal of the cell expansion phase is to achieve optimal cell concentration at the transfection day. In one embodiment the cells are expanded up to obtain a concentration between 0.9x10⁵ cells/cm² and 2x 10⁵cells/cm² of the surface area of the bioreactor. Cell counting in the packed bed bioreactor system is not always practical and possible therefore, following the inoculum, cell expansion can be monitored by measuring appropriate metabolic parameters such as lactate accumulation or glucose consumption. In a preferred embodiment the cells are cultivated for expansion until obtaining an accumulation of lactate into the bioreactor between 0,7 g/l to 1g/I, In a further preferred embodiment the cells are inoculated at day 0 into the bioreactor and then are cultivated up to day 4 or, most preferably up to day 3.

In one embodiment the cells are inoculated into at day 0 into the bioreactor and then are cultivated by operating a cell culture in batch mode. In a preferred embodiment the cells are inoculated at day 0 into the bioreactor and then are cultivated in batch mode up to day 4 or, most preferably up to day 3. Preferably, at the end of cultivation for expansion in batch mode, the exhausted cell culture medium is changed before transduction. In one embodiment the exhausted cell culture medium is changed by draining the bioreactor and adding fresh medium. In another embodiment the exhausted cell culture medium is changed in perfusion mode by supplying at fix rate the fresh medium in an amount corresponding to at least the same volume of the cell culture medium present in the bioreactor.

In another embodiment the cells are inoculated at day 0 into the bioreactor and then are cultivated in perfusion mode up to day 4 or, more preferably up to day 3. Perfusion may be activated after at least four hours from cell inoculum.

In a preferred embodiment the cells are inoculated at day 0 into the bioreactor and then are cultivated in re-circulation mode up to day 4 or, more preferably up to day 3. Perfusion may be activated after at least four hours from cell inoculum.

Many basic culture media are known in the art that can be suitable for the method of the invention. Culture media include but are not limited to EMEM (Eagle Minimum Essential Medium) BEM (Basic Eagle Medium), DMEM (Dulbecco's modified Eagle Medium), Glasgow minimal essential medium, M199 basal medium, HAMs F-10, HAMs F-12, Iscove's DMEM, RPMI, Leibovitz L15, MCDB, RPMI-1640, IMDM, X-Vivo10^{™}, X-Vivo15^{™} and X-Vivo20^{™} . Preferably, the cell culture medium is supplemented with other factors known to promote cell expansion such as glutamine or glutamax, Fetal bovine serum (FBS) or alternatives that can easily be determined by the skilled person.

### Transfection step

In the method of the present invention the cells are transfected with two or more plasmid(s) (i.e.: a multi-plasmid DNA transfection) adapted for the production of a lentiviral vector.

Various techniques known in the art may be employed for introducing nucleic acid molecules into cells. Such techniques include chemical- facilitated transfection using compounds such as calcium phosphate, cationic lipids, cationic polymers, liposome-mediated transfection. However, according to a preferred aspect of the invention multi-plasmid DNA transient transfection is carried out using polyethylenemine (PEI) as a transfection reagent.

The manufacturing method according to the present invention comprises a transfection step in which the transfection is started by adding into the bioreactor the transfection mix.

As used herein the term transfection mix means a solution containing the DNA transfecting particles formed further to the mixing of the DNA with the transfecting agent.

Preferably, the preparation of the transfection mix includes the following steps:
a) Preparing of a solution containing the DNA (solution a)
b) Preparing a solution containing the PEI (solution b)
c) Mixing the two solutions a and b, and further incubating them to obtain a transfection mix containing the DNA transfecting particles.

The transfection is performed using a total amount of multi-plasmid DNA between 45ng/cm² and 100ng/cm² of the surface area of the packed bed.

In the method of the invention, the transfection is performed using a ratio between DNA amount and PEI equal to 1:1.

According to the method of the invention, transfection is performed by adding into the bioreactor the transfection mix containing DNA transfecting particles and incubating the cells into the bioreactor with the DNA transfecting particles in absence of perfusion or recirculation of fresh medium.

In one embodiment the transfection mix is added to the cell culture medium of the bioreactor following to, where necessary, the removal of a volume of cell culture medium proportional or equal to the volume of the transfection mix.

In the method of the invention during transfection, the incubation of the cells with the DNA transfecting particles is performed in absence of perfusion or recirculation of fresh medium so as to maintain constant the quantity of the DNA transfecting particles into the bioreactor. Application of stirring is maintained to permit O2 supply and circulation of the DNA particles.

As shown in the examples, the incubation time with the DNA transfecting particles may have impact on the productivity and quality of the final vector preparations. Examples 2.3 and 2.4 show that LVVs obtained further to 21 hours of incubation with the DNA transfecting particles have a reduced infectivity. Therefore, according to a preferred aspect of the invention, during transfection the cells are incubated with the DNA transfection particles for a period up to 18 hours. In addition, as shown in the Example 1.3, a decrease in productivity can be observed by reducing incubation time with the DNA transfecting particles to 6 hours. The productivity, in this condition is still acceptable, but a further reduction of incubation time is expected to be even less effective. Therefore, in a preferred embodiment the cells are incubated with the DNA transfecting particles for a period between 6 and 18 hours, more preferably for a period of 8 hours.

### Optimal total DNA quantity in transfection

As explained above, in the method of the invention the cells are transfected with two or more plasmids encoding the required components necessary to produce the vectors (multi-plasmid DNA transfection). Different protocols are known in the art disclosing different ratios of each plasmid to be used for transfection. Depending on the plasmid system used (e.g.: number of packaging plasmid used, nature and length of the transfer vector plasmid) the skilled man may optimize the optimal ratio between the different plasmids required for LVV production. Once defined, for examples in flasks or wells, such ratio has to be applied to the total quantity of DNA to be used in the bioreactor system.

The inventors identified the ideal range of total multi-plasmid DNA to be used in the method of the invention in order assure high productivity and reproducibility of the process.

In one embodiment the transfection is performed using multi-plasmid DNA in a total amount of DNA between 45ng/cm² and 100ng/cm² of the surface area of the packed bed.

The inventors surprisingly found that, it was possible to use the DNA in a range of quantity lower than that disclosed in prior art, obtaining bulk productions having comparable or even higher productivity.

Prior art (Valkama et al. 2018 and Leinonen et al 2019) discloses to use in transfection of total DNA in an amount of 200 ng/cm² or 300 ng/cm² or 400 ng/cm² with PEI as transfection reagent (ratio DNA:PEI equal to 1:1). Moreover, Valkama et al 2018 and WO 2018/ 007873 clearly states that by using in transfection the DNA in an amount of 100ng/cm² transfection efficiency is reduced to 5%. Example 1.2 of the present invention shows the results obtained by performing a manufacturing method for the production of LVVs viral vectors in packed bed bioreactor by multi-plasmid DNA transient transfection performed using different DNA total amount i.e.: 363ng/cm², 181 ng/cm², and 91 ng/cm². Table 4 clearly shows that the bulk productions obtained using in transfection total DNA in an amount of 181 ng/cm² and 91 ng/cm² result to have higher productivity (higher physical titer) compared to runs obtained with 363 ng/cm² of total DNA. Such surprising effect is also observed by applying the process in iCellis nano having 1,06 m² surface area as shown in example 2.3 and related table 23, in which it was found that the bulk production obtained using 91 ng/cm² of total DNA have comparable productivity in respect to that obtained by transfecting cells with 181 ng/cm²of total DNA. Consistent data were obtained in large scale as shown in example 3. Contrary to what is reported in prior art, by reducing the amount of total DNA used in transfection below 200ng/cm² of the surface area it was possible to achieve comparable or even higher productivity. The results were observed with DNA amounts even lower than that recommended by the PEI manufacturer for a single plasmid transfection (at least 100 ng/cm² of the surface area). This is particularly important taking into consideration that, in the process of the present invention it is necessary to co-transfect different plasmids in the cells to obtain production of LVVs.

Mc Carron et al . 2019 disclose a manufacturing processes in packed bed bioreactor in which a 5 plasmids lentiviral vector system is used. Co-transfection, in this case, is performed using 26,5 ng/cm² of total DNA and PEI transfection reagent (ratio DNA:PEI 1:3, i.e.: using an amount of PEI three times higher than that used in the examples). The bulk production obtained was described to have a low yield 1X10⁷ vp/cm² corresponding to 1 ng/cm² of p24 measured i.e.: a titer up to about more than 70 times lower than that observed by using DNA in the range according to the present invention.

Therefore, according to the present invention, there is provided a method for the manufacturing of lentiviral vectors in a packed bed bioreactor comprising transiently transfecting the cells using multi-plasmid DNA in a total amount between 45ng/cm² and 100ng/cm² of the surface area of the packed bed and the PEI transfection reagent.

According the invention there is provided a method the for manufacturing of lentiviral vectors in a packed bed bioreactor by multi-plasmid transient DNA transfection comprising the following steps:
(a) Inoculating the cells
(b) Culturing the cells for expansion for at least one day
(c) Transfecting the cells using multi-plasmid DNA in a total amount between 45ng/cm² and 100ng/cm² of the surface area of the packed bed and the PEI transfection reagent
(d) Harvesting the cell culture supernatant containing the recombinant lentiviral vectors The transfection is performed using a ratio DNA:PEI equal to 1:1

In a preferred embodiment the multi- plasmid DNA is composed by one plasmid carrying the lentiviral gag/pol genes, one plasmid carrying lentiviral rev gene, one plasmid carrying the gene encoding the envelope protein, preferably the gene encoding the VSV-G envelope protein, and one plasmid carrying the transfer vector including the required portion of the lentiviral genome as disclosed above and the gene foreign gene of interest.

### Post transfection change of medium

As disclosed above, it is necessary to monitor and measure the transfection time i.e.: the period in which the cells are incubated with the transfection particles as well as to maintain a constant quantity of transfecting DNA particles in the bioreactor during the incubation time. The manufacturing method according to the present invention includes the change of medium at the end of transfection (i.e. post transfection). Such step is effective for the removal, at the end of the incubation time, of the DNA transfecting particles, that may negatively affect the productivity of the process and the quality in terms of contaminants present in the bulk production. The change of medium in a packed bed bioreactor system may be performed in perfusion mode by supplying, at fix rate, a sufficient amount of fresh medium. For example it is possible to supply at fix rate an amount of medium corresponding to the same or the double total volume of medium used in the bioreactor during transfection. Alternatively, the post transfection change of medium may be performed in batch mode i.e. by draining from the bioreactor the cell culture medium containing the DNA transfecting particles and adding the fresh medium to the cells. The step of post transfection medium change, thanks to the removal of the DNA transfecting particles, allows to stop the transfection, thus permitting to control the time of transfection i.e.: the period in which the cells are incubated with the transfection particles that, as explained above may also influence the infectious viral titers of the bulk production.

In a preferred configuration, the method of the invention includes a change of medium performed in batch mode by draining from the bioreactor the cell culture medium containing the DNA transfecting particles and adding the fresh medium to the cells. In one embodiment up to 98% of the cell culture medium containing containing DNA transfecting particles is removed from the bioreactor. Alternatively up to about 95% or up to about 90% or up to about 85% or up to about 80% or up to about 75% or up to about 70% of the cell culture medium containing containing DNA transfecting particles can be removed from the bioreactor.

Prior art discloses to perform a post transfection complete change of medium in perfusion mode. Valkama et al. 2018 disclose a method in which perfusion is applied to the packed bed bioreactor after 4 or 6 hours of transfection and, following, a total change of medium in perfusion is performed one or two days after transfection during the collection of LVVs by perfusion. Leinonen et al 2019 disclose a change of medium performed by applying perfusion after transfection until complete change of medium at 24 hours post transfection, before starting collection of LVVs particles by perfusion. Mc Carron et al. 2019 disclose post transfection change of medium in batch mode, followed by collection of LVVs in batch mode. Prior art does not disclose post transfection change of medium in batch mode combined with collection LVVs in perfusion mode. As shown in the examples it was surprisingly found that application of perfusion to perform post transfection change of medium is detrimental for the productivity (physical and infectious viral titer) of the bulk production. In particular, Example 1.5 and related table 13 show that LVVs produced with a method according to the present invention (draining of cell culture medium from the bioreactor and addition of the fresh medium to the cells followed by harvesting in perfusion) have higher physical titer and higher infectious viral titer in respect to those obtained with a process in which post transfection medium change is performed by applying perfusion until total change of medium as disclosed in prior art. Moreover, as reported in table 14 the change of medium post transfection in batch mode results to be effective in the reduction of contaminants (plasmid DNA, total DNA and HCP) in the final pool without treatment with endonuclease.

This technical effect is not only surprising by itself, it is also totally unexpected in consideration that, at large scale, the total draining of the bioreactor and the subsequent filling require a certain time (approximately 50 minutes) in which the cells remain without cell culture medium. Such technical limitation was expected to cause problems to the production, as logical consideration and also in view of prior art teaching. WO 2018/ 007873 for example states: *".... at large scale the full exchange of medium is not practical process step because it requires time and may be influencing on cell viability due to the fact that during draining, stirring is closed and the cells in the upper carrier are without medium".*

On the contrary, data in the application show that there is no indication of a potential detrimental effect on the productivity and infectious viral titer caused by the total removal in batch of the cell culture medium at large scale. In example 1.5, one of the manufacturing runs performed includes and holding time of 50' after draining of the cell culture medium during post transfection medium change in batch mode, in order to mimic the emptying/filling timing of the large scale. Results obtained by performing the run including the 50' holding time confirm an increase of productivity and infectious viral titer in respect to a production run including post transfection medium change in perfusion mode.

Moreover, example 3 shows the results obtained by performing the manufacturing method according to the present invention in large scale. In order to evaluate the efficiency of the process in large scale a run in small scale (iCellisNano) was performed in parallel with the run in iCellis500 and data in terms of physical titer and infectious viral titer were directly compared. The productivity (as physical and infectious viral titer) and the quality of final product (in terms presence of contaminants) obtained in large scale are consistent with or even better than those obtained in small scale runs performed in parallel (as shown for example for physical infectious viral titer and infectivity as well as presence of contaminants shown in Example 3.2).

### Collection of LVV recombinant particles

The identification of the harvesting operational procedure and period for the collection of LVV recombinant particles is one of the most important goals for the development of an effective manufacturing process. According the method of the present invention, the collection of the recombinant LVVs is performed by starting the harvest of the cell culture medium containing the recombinant LVVs (also herein referred as cell culture supernatant) in perfusion mode, immediately after the post transfection medium change performed in batch as disclosed above. Prior art discloses methods for production of LVVs using packed bed bioreactor in which all steps are performed in batch (such as Mc Carron et al. 2019) or in which all steps are performed in perfusion (such as in Valkama et al 2018 or Leinonen et al 2019). In the method according to the present invention, the inventors found that following transfection, a combination of steps performed in batch with steps performed in perfusion results to be successful for the performance of an effective and reproducible manufacturing process.

In Mc Carron et al. 2019 recombinant LVVs particles are collected in batch after post transfection change of medium performed in batch. The cell culture medium in the bioreactor is removed for LVVs collection at 48 hours post transfection and at 72 post transfection, to permit the maximum accumulation of the recombinant LVVs particles in the cell culture medium. An increase of productivity is observed at 72h post transfection and the authors suggest to further evaluate the productivity after 72 hours post transfection. The total time required for transfection and subsequent harvesting is 4 days.

In Valkama et al 2018 or Leinonen et al 2019 collection is performed in perfusion after 24 h from transfection, in both papers there is a clear indication of very low production of LVVs 6 hours after transfection. Moreover, in Leinonen et al 2019 the authors states that post transfection change of medium (performed in perfusion) causes a reduction in productivity, for this reason certain additives are added in the medium used for harvesting.

In the method according to the present invention, the performance of post transfection change of medium in batch followed by immediate starting of LVVs collection by harvesting in perfusion allows to avoid the reduction of productivity observed in prior art.

In one embodiment according to the method of the invention harvesting in perfusion is performed for 48 hours after the post transfection medium change.

In a preferred embodiment according to the method of the invention harvesting in perfusion is performed for at least 39 hours after the post transfection medium change.

In a more preferred embodiment there is provided a method according to the present invention wherein the steps of transfection, post transfection medium change and harvesting in perfusion have a total duration of three days.

Particularly, example 2.4 shows the results obtained with an optimized version of the manufacturing method according to the present invention. The example compares the results obtained by performing transfection and subsequent harvesting in 3 days (8 hours of transfection followed by medium change in batch and 39 hours of harvesting in perfusion) with those obtained by performing transfection and subsequent harvesting in 4 days (21 hours of transfection followed by medium change in batch and 48 hours of harvesting). As shown in table 27 infectious viral titer and infectivity are higher when media change post transfection and final harvest are anticipated by one day i.e.: when transfection and subsequent harvesting are performed in three days. Moreover, pool harvests obtained performing transfection and harvesting in a total of three days results to have less contaminants with total DNA being undetectable or below the analytical method limit without endonuclease treatment.

### KEY ADVANTAGES OF THE INVENTION

The present invention relates to a method for the manufacturing of LVVs in a packed bed bioreactor, in which the final bulk productions are characterized by high productivity in term of physical titer, infectious viral titer and infectivity and low quantity of contaminants. Particularly, the inventors selected a combination of operational conditions to perform certain steps of the process, that positively affect the productivity of the process. Surprisingly, the post transfection change of medium performed in batch mode results to have a positive effect on the productivity in terms of physical titer and infectious viral titer as directly compared with a post transfection change of medium performed in perfusion mode. As disclosed above, prior art (WO 2018/ 007873) suggests to avoid draining of cell culture medium from the bioreactor particularly at large scale. On the contrary, data shown in examples 3 prove the efficacy of the process performed in large scale and the reproducibility of the results obtained in small scale with no negative impact of the post transfection medium change performed in batch mode. Example 3.2 shows that by performing two manufacturing runs in parallel one on iCellis 500 (large scale surface area 133m²) and another in iCellis nano (small scale 1,06 m²) the productivity in large scale results to be higher with the infectious viral titer and the infectivity of bulk productions in large scale double than that obtained in small scale. Moreover, the combination of post transfection change of medium with LVVs collection by harvesting in perfusion allows to obtain a final bulk production characterized by a very good purity profile in terms of total DNA content and host cell protein contents in small scale as well as in large scale. As shown in the example 2.4, total DNA and host cells proteins contaminants in the bulk preparations are minimal or even undetectable without any treatment with endonuclease. Results are confirmed in large scale as shown in example 3.2. This is a further surprising effect, considering that prior art discloses the need to perform a first endonuclease treatment in the bioreactor during the production phase of the process (i.e. during the collection of LVVs) followed by a further endonuclease treatment of the bulk preparation in order to achieve acceptable results in terms of purity.

Moreover, the inventors identify an effective range of total DNA quantity to be used for transfection in the bioreactor that allows to obtain productivity comparable or even higher in respect to that obtained with larger quantities of total DNA in transfection. Such effect is already observed by operating the process totally in batch mode, with further improvements by applying more effective combination of operational methods as disclosed above. The reduction of total amount of DNA while maintaining high productivity, is an important advantage in the economy of the process since, large scale manufacturing of LVVs for pharmaceutical preparations require the use of GMP grade raw materials and DNA plasmids are among the most expensive materials, therefore, there is a significant positive impact on the costs. For example, data reported in example 3.2 show that by performing a production run in large scale in iCellis 500 using in transfection multi-plasmid DNA in total quantity of 91ng/cm2 it was possible to obtain a bulk preparation having physical titer of 60ng/cm², infectious viral titer doubled in respect to that obtained with the same process at small scale and low impurity profile. Data show the reproducibility and effectiveness of the process at small as well as at large scale. Moreover, data reported in example 4 show that by further reducing the total quantity of multi-plasmid DNA used in transfection to 60 ng/cm2 or 45 ng/cm2, productivity in terms of physical titer is substantially maintained and a good level of infective viral particles is observed.

### DESCRIPTION OF THE DRAWINGS

Figure 1: flow chart representing the processes performed in the experiments disclosed in Example 1.
Figure 2: flow charts representing the processes performed in the experiments disclosed in Example 2: (*) Recirculation was applied from day 1 to day 3 for run PDE_B18172 and from day 0 to day 3 from run PDE_B18187 on; (**) Condition applied only for run PDE_B18172. (***) Conditions applied from run PDE_B19013 on, except for bio2 of run PDE_B19087 (media change 6h after transfection).

### EXAMPLES

### EXAMPLE 1 - DESCRIPTION OF LENTIVIRAL VECTOR PRODUCTION IN iCELLis NANO 0,53 m²

### EXAMPLE 1.1 - MATERIALS AND METHODS

### PACKAGING CELLS AND CELL CULTURE MEDIUM

Lentiviral vector is produced by HEK 293T cultured in IMDM without phenol red, 10% FBS and 2% L-Glutamine or 2% Glutamax).

### BIOREACTOR

During feasibility study, all the experiments described in this Example 1 have been done in iCELLis nano with the characteristics reported in Table 1.

**Table 1: iCellis nano characteristics**

| **Bioreactor Size (m²)** | **FB height (cm)** | **Compaction** | **Carrier Density (g/L)** | **No. of carriers** |
|---|---|---|---|---|
| 0.53 | 2 | 1 | 96 | 472 |

### PLASMIDS

The multi plasmid DNA system used is a third generation LVVs packaging system including 4 separate plasmids: one plasmid carrying gag-pol genes (pGag/Pol), one plasmid encoding rev (pREV), one plasmid carrying VSV-g gene (pENV-VSV-G)), and one transfer vector plasmid carrying the gene encoding GFP protein (pTransfer-GFP).

### ANALYTICAL METHODS

The following analytical methods have been applied to evaluate process performance:
- Physical viral titer: this analytical method is an ELISA to quantify p24 HIV protein, which can be used to evaluate the concentration of physical particles.
- Infectious Viral Titer: this analytical method is based on transduction of reference CEM A3.01 cell line. The expression of the GFP protein is evaluated by FACS analysis.
- Residual Host Cell Proteins: this analytical method is an ELISA to quantify residual proteins from HEK-293T packaging cell line in LVV samples.
- Residual Total DNA: this analytical method is based on a fluorescent dye that stains and allows quantification of residual DNA.

### SUMMARY OF THE MANUFACTURING PROCESS

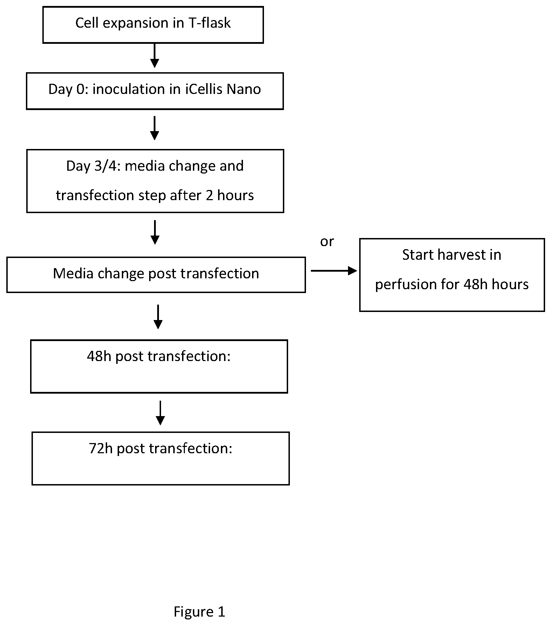

A flow chart of the processes applied in experiments disclosed in Example1 is reported in Figure 1. Briefly, for each experiment cells were expanded in flasks in IMDM w/o phenol red and containing 10% FBS and 2% L-glutamine (or glutamax). After split, cells were inoculated in the bioreactor iCELLis nano (5000 cells/cm2). After expansion phase in which pH, DO%, glucose and lactate concentration were monitored, the culture medium was changed with fresh complete medium and transfection step was performed at day 3 or 4. After 18 hours, a new media change was performed in order to remove every residuals of plasmid DNA and PEI Pro. Two harvests have been collected at 48h and 72h after transfection step. The lentiviral vector from each harvest was filtered 0.45µm and stored at <-65°C in samples for testing. From run PDE_B17054 B in the experiments shown in this example 1, harvest was performed in perfusion mode and the LV vectors were collected after 48 h of perfusion.

### TRANSFECTION STEP

In all the experiments disclosed in this Example 1, production of LV vectors was performed using Polyethylenimine (PEI)- mediated transfection method, that is easy-to-use, with a high degree of reproducibility and consistency. In order to identify the appropriate amount of plasmid DNA to be used in the PEI transfection protocol, the feasibility study was performed starting from the amount resulting functional in other cell culture systems (such as flasks) and consistent with amount disclosed in prior art (i.e.: from 300 ng/cm2 to 400 ng/cm2 of surface area as disclosed in Valkama et al 2018 or from 200 to 300 ng/cm2 as disclosed in Leinonen et al 2019). The amount of PEl used has been set to maintain a ratio of 1:1 between PEI and DNA.

Protocol of transfection:
- Perform medium change with pre-warmed complete medium 1 hour before transfection.
- Prepare a DNA mix, diluting the correct amount of the plasmid DNA in IMDM free.
- Dilute the correct amount of PEI in IMDM free
- Transfer the diluted PEI in the tube containing the diluted DNA and mix.
- Incubate the final mix (100 mL for 1 bioreactor) according to the manufacturer instructions.
- Transfer the mix into into the bioreactor.

### EXAMPLE 1.2 - AMOUNT OF TOTAL DNA

In this first group of experiments the following conditions were applied for the performance of manufacturing runs in iCellis Nano Bioreactor having a surface area of 0,53 m²:
- Cell seeding density: 5000 c/cm²;
- Media change post transfection in batch at 18h;
- 2 harvests performed at 48h and 72h after transfection.

The optimal amount of DNA for transfection have been investigated. The transfection has been performed at day 3 after the inoculum for every run. In run PDE_M16142, PDE_M16155 Bio1 and PDE_M16177 the transfection was performed using DNA in a total amount of 181 ng/cm². In order to investigate different ratio, the double amount of DNA (2x) has been used in run PDE_M16121 Bio1 i.e.: 363 ng/cm² (consistent with DNA quantity disclosed in prior art) and the half amount (1/2) in run PDE_M16155 Bio2 i.e: 91 ng/cm². The different conditions used are summarizes in Table 2.

**Table 2: Experimental conditions**

| **Process ID** | **DNA (ng)/surface area (cm²)** | **Total amount of plasmid DNA (µg)** |
|---|---|---|
| **PDE_M16121 Bio1** | 363 | 1925 |
| **PDE_M16142** | 181 | 962 |
| **PDE_M16155 Bio1** | 181 | 962 |
| **PDE_M16155 Bio2** | 91 | 481 |
| **PDE_M16177 Bio2** | 181 | 962 |

Briefly, in all the experiments, 5000 cells/cm² were inoculated in iCELLis nano. Sampling have been performed from day 3 to day 6 in order to monitor cell growth, measuring pH, lactate and glucose concentration. During all the experiments, at day 3 before transfection, three microcarriers were taken from each bioreactor and lysated; a nuclei count was performed in order to estimate the cell density for cm²; the exhausted culture medium was removed and changed with fresh medium. PEl-mediated transfection was performed according to the protocol described previously (Example 1.1). 18 hours after transfection the exhausted culture medium was removed (drained from the bioreactor) and changed with fresh medium for production. Two harvests in batch were performed 24h, 48h after media change.

The number of cells for cm² at the transfection day and lactate produced (mg/mL) from day 3 to day 6 are reported in Table 3.

**Table 3: Cell density (cells/cm²) and lactate (mg/mL) data**

| **Process ID** | **Cell density** | **Lactate (mg/mL)** | | | |
|---|---|---|---|---|---|
| | **n. cells/cm²** | **Day 3** | **Day 4** | **Day 5** | **Day 6** |
| **PDE_M16121 Bio1** | 1.22E+05 | 0.745 | 0.756 | 0.737 | 0.865 |
| **PDE_M16142** | 1.37E+05 | 0.756 | 1.11 | 1.02 | 1.22 |
| **PDE_M16155 Bio1** | 0.98E+05 | 0.832 | 1.17 | 1.04 | 1.25 |
| **PDE_M16155 Bio2** | 1.15E+05 | 0.847 | 1.25 | 1.2 | 1.48 |
| **PDE_M16177 Bio2** | 1.02E+05 | 0.746 | 1.1 | 1.0 | 1.31 |

The results are summarized in Table 4:

**Table 4: Productivity data**

| **Process ID** | **Physical viral titer** | | | **Infectious viral titer** | | | **Infectivity** |
|---|---|---|---|---|---|---|---|
| | **ngP24/mL** | **tot ngP24** | **ngP24/ cm²** | **TU/mL** | **Total TU** | **TU/cm2** | **TU/ngP24** |
| **PDE_M16121 Bio1** | 134 | 2.41E+05 | 45.5 | 8.0E+06 | 1.44E+10 | 2.72E+06 | 5.97E+04 |
| **PDE_M16142** | 345 | 6.21E+05 | 117.2 | 1.5E+07 | 2.70E+10 | 5.09E+06 | 4.35E+04 |
| **PDE_M16155 Bio1** | 289 | 5.20E+05 | 98.2 | 9.0E+06 | 1.62E+10 | 3.06E+06 | 3.11E+04 |
| **PDE_M16155 Bio2** | 163 | 2.93E+05 | 55.4 | 6.0E+06 | 1.08E+10 | 2.04E+06 | 3.68E+04 |
| **PDE_M16177 Bio2** | 262 | 4.72E+05 | 89.0 | 1.00E+07 | 1.80E+10 | 3.40E+06 | 3.82E+04 |

As the results show, bulk productions obtained using in transfection total DNA in an amount of 181 ng/cm² and 91 ng/cm² result to have higher productivity compared to runs obtained with 363 ng/cm2 of total DNA. For this reason, in the following runs, 962 µg have been used for transfection step.

### EXAMPLE 1.3 - INCUBATION OF THE TRANSFECTION MIX

In the following group of experiments, the transfection was performed at day 3 in iCellis Nano Bioreactor having a surface area of 0,53 m² using multi-plasmid DNA in total amount of 962µg per bioreactor (i.e.: 181ng/cm² of surface area) and PEI PRO (1µg/µl) in total amount of 962µg. In run PDE_B17028 A, media change was performed 6h after the starting of the transfection phase, in order to investigate if an earlier medium change was associated to enhanced LVV productivity. In order to reduce the amount of FBS used in the process, in run PDE_M17028B a reduction of percentage of serum (2.5%) during the production phase was investigated.

The different tested conditions are indicated in Table 5.

| Table 5: Experimental schedule**Process ID** | **% of FBS** | **Media change post transfection** |
|---|---|---|
| **PDE_M16203 A** | 10 | 18h |
| **PDE_M17028 A** | 10 | 6h |
| **PDE_M17028 B** | 2.5 | 18h |

5000 cells/cm² were inoculated in iCELLis nano at day 0. Sampling have been done from day 3 to day 6 in order to monitor cell growth; at day 3 before transfection, three carriers were taken from each bioreactor and lysates; a nuclei count was performed in order to estimate the cell density for cm². The exhausted culture medium was removed, changed with new growing medium and Pei-mediated transfection was performed;. 18 h after transfection step (6 hours for run PDE_M17028A), exhausted medium change in batch mode was performed. In run PDE_M17028 B the exhausted media was replaced with IMDM 2.5%FBS and 2% L-glutamine. As in the previous runs, two harvests in batch were performed 24h and 48h after media change.

The results of physical and infectious viral titer and infectivity of pool harvest are summarized in Table 6.

**Table 6: Productivity data**

| **Process ID** | **Physical viral titer** | | | **Infectious viral titer** | | | **Infectivity** |
|---|---|---|---|---|---|---|---|
| | **ngP24/mL** | **tot ngP24** | **ngP24/cm²** | **TU/mL** | **Total TU** | **TU/cm2** | **TU/ngP24** |
| **PDE_M16203 A** | 271 | 4.88E+05 | 92.0 | 1.0E+07 | 1.80E+10 | 3.40E+06 | 3.69E+04 |
| **PDE_M17028 A** | 155 | 2.79E+05 | 52.6 | 3.0E+06 | 5.40E+09 | 1.02E+06 | 1.94E+04 |
| **PDE_M17028 B** | 293 | 5.27E+05 | 99.5 | 8.0E+06 | 1.44E+10 | 2.72E+06 | 2.73E+04 |

The results of the run PDE_M17028 A showed that an earlier medium change after transfection (6 hours of incubation of cells with the transfection mix) decreased the production of the vector; reduction of percentage of FBS during the harvest phase (PDE_M17028 B) had no impact on production, but reduced viral titer and infectivity of the LV vector.

### EXAMPLE 1.4 COLLECTION OF LVVS BY HARVESTING IN PERFUSION

In order to increase the infectivity of viral vector produced and avoid the emptying/filling time between harvests of iCELLis 500, in run PDE_B17133, harvest in perfusion was evaluated and compared with harvest in batch. In detail, PDE_B17133 A was performed with two harvests in batch, while in run PDE_B17133B LV vector was harvested in perfusion mode. Both bioreactors followed the same process until transfection phase. Briefly, two bioreactors were inoculated with 5000 Cells/cm² at day 0. After three days, medium change pre-transfection with complete medium was performed; followed by transfection with the same protocol followed in the previous runs. After 18 hours, media change post-transfection in batch was performed with new complete medium. Sampling have been done from day 3 to day 6 in order to monitor cell growth; before transfection, three carriers were taken from each bioreactor and lysate to perform nuclei count.

In PDE_B17133 A, the supernatant was collected in perfusion with at fix rate (13.5 mL/hour rate): fresh medium was transferred into an IN bottle, connected with the bioreactor and perfused in 48 hours. During harvest in perfusion, supernatant was collected in an OUT bottle. Samples from the OUT bottle were collected at 24h and 48h after media change, filtered 0.45 µm and stored at <-65°C. At 48h, supernatant inside bioreactor and in OUT bottle were pooled, filtered and stored at <-65°C. Samples from pool were collected, filtered 0.45 µm and stored at <-65°C.

In PDE_B17133 B, two harvests were collected in batch at 24h and 48h after media change post transfection. Samples from each harvest were collected and stored at <-65°C. At the end of the process, the two harvest were pooled, filtered and stored at <-65°C.

In Table 7 are summarizes the conditions used for the two bioreactors.

**Table 7: Experimental schedule of processes PDE_B17133A and PDE_B17133B**

| **Day** | **PDE_B17133A** | **PDE_B17133B** |
|---|---|---|
| **0** | Seeding at 0.5x10⁴/cm² | |
| **1** | Expansion phase | |
| **2** | | |
| **3** | Media change pre-transfection | |
| | PEl-mediated transfection step (as disclosed in example 1.3) | |
| **4** | Media change post-transfection | |
| | Start perfusion: 13.5 mL/h | n.a. |
| **5** | Harvest in perfusion in 48 hours | 1' harvest |
| **6** | | 2' harvest |

Results are reported in the Table 8.

**Table 8: Productivity data**

| **Process ID** | **Physical viral titer** | | | **Infectious viral titer** | | | **Infectivity** |
|---|---|---|---|---|---|---|---|
| | **ngP24/ mL** | **tot ngP24** | **ngP24/ cm²** | **TU/mL** | **Total TU** | **TU/cm2** | **TU/ngP24** |
| **PDE_B17 133 A** | 168 | 2.18E+05 | 41.00 | 8.0E+06 | 1.04E+10 | 1.96E+06 | 4.76E+04 |
| **PDE_B17 133 B** | 179 | 2.33E+05 | 43.00 | 8.0E+06 | 1.04E+10 | 1.96E+06 | 4.47E+04 |

The results demonstrated that the physical and infectious viral titer and infectivity obtained from the two bioreactors were comparable. The condition that has been introduced (supernatant harvested in perfusion started after post transfection change of medium in batch) allows to maintain the same level of productivity of the harvest in batch .

### EXAMPLE 1.5 CULTURING CONDITIONS PRE-TRANSFECTION

In order to optimize the scale up in iCellis 500 and avoid the emptying/filling time during all phases of the process, an alternative to media change pre-transfection in batch was evaluated. In particular, in runs PDE_B18034 and PDE_B18068, media change in perfusion and in recirculation were introduced. The Table 9 shows the investigated conditions in each run.

**Table 9: Experimental schedule for Group #9**

| **Process ID** | **Medium change pre transfection** | **Volume of medium change** |
|---|---|---|
| **PDE_B18034 A** | Perfusion | 1300 mL |
| **PDE_B18034 B*** | Batch | 650 mL |
| **PDE_B18068 A** | Recirculation | 650 mL |
| **PDE_B18068 B** | Perfusion | 1300 mL |
| **PDE_B18068 C** | Perfusion | 650 mL |

Briefly, each bioreactor iCellis nano was inoculated at day 3 with 5000Cells/cm² and sampling have been done from day 3 to day 6 to monitor pH, lactate and glucose.

At day 3, medium change pre-transfection was performed as explained below:
- PDE_B18034 A and PDE_B18068 B: exhausted medium has been changed with new growing medium in perfusion mode. Perfused volume was 1300 mL.
- PDE_B18034 B: medium change was performed in batch; exhausted medium was removed and bioreactor was filled with 650 mL of new growing medium.
- PDE_B18068 C: exhausted medium has been changed with new growing medium in perfusion mode. Perfused volume was 650 mL.

PDE_B18068A: in this run media change was not performed, but at day 1 after inoculum, recirculation started with 650 mL of fresh medium, until day 3.

PEl-mediated transfection was performed as disclosed in example 1.3. From transfection phase to the end of the process, the harvest in perfusion for 48 hours has been performed in the three bioreactors During perfusion phase, samples have been collected at 24h and 48 h post media change from OUT bottle in order to measure physical and infectious viral title.

Total pool from PDE_B18034 and PDE_B18068 were tested for physical and infectious viral title.

The results of physical and infectious viral title and infectivity of the experiments are reported in Table 10:

**Table 10: Productivity data**

| **Process ID** | **Physical viral titer** | | | **Infectious viral titer** | | | **Infectivity** |
|---|---|---|---|---|---|---|---|
| | **ngP24/mL** | **tot ngP24** | **ngP24/cm²** | **TU/mL** | **Total TU** | **TU/cm2** | **TU/ngP24** |
| **PDE_B18034 A** | 239 | 3.11E+05 | 58.00 | 8.0E+06 | 1.04E+10 | 1.96E+06 | 3.35E+04 |
| **PDE_B18034 B** | 170 | 2.21E+05 | 41.00 | 4.0E+06 | 5.20E+09 | 9.81E+05 | 2.35E+04 |
| **PDE_B18068 A** | 211 | 2.74E+05 | 51.00 | 8.0E+06 | 1.04E+10 | 1.96E+06 | 3.79E+04 |
| **PDE_B18068 B** | 259 | 3.37E+05 | 63.00 | 9.0E+06 | 1.17E+10 | 2.21E+06 | 3.47E+04 |
| **PDE_B18068 C** | 212 | 2.76E+05 | 52.00 | 9.0E+06 | 1.17E+10 | 2.21E+06 | 4.25E+04 |

The results demonstrated that the productivity of LV vector is higher in runs in which cell culture for expansion is performed with media change in perfusion or recirculation mode, if compared with standard condition (run PDE_B18034B, medium change performed in batch).

### EXAMPLE 1.5 POST TRANSFECTION CHANGE OF MEDIUM

The goal of this group of experiments was the study of the best technical solution to perform the medium change post transfection.

In detail, in run PDE_B18080 A, medium change post transfection was performed in perfusion in order to avoid the emptying and filling of the vessel (laborious and difficult in iCellis 500 and discouraged in prior art such as WO 2018/ 007873). 18h after transfection, 1300 mL of complete fresh IMDM were perfused with a ratio of 9 mL/min in order to exchange 90% of medium.

In run PDE_B18080 B, holding time of 50' was introduced during medium change post transfection in batch in order to mimic the emptying/filling timing of iCELLis 500. 18 hours after transfection phase, exhausted medium was removed and bioreactor was left empty for 50' leaving only 16 mL inside it; this amount of medium correspond to the volume that cannot be recovered in iCellis 500 during the empting due to technical limitation of the system. After 50' bioreactor was filled with new fresh medium.

In PDE_B18080C, medium change post transfection was performed with standard condition (batch, without holding time) in order to compare it with the new conditions introduced.

Samples have been done from the three bioreactors before and after medium change post transfection in order to evaluate plasmid residual DNA. In bioreactor A, samples have been done from the vessel after the half volume perfusion.

Different medium change conditions are summarized in Table 11.

**Table 11: Medium change conditions tested**

| **Process ID** | **Medium change post transfection** | **Holding time** |
|---|---|---|
| PDE_B18080 A | Perfusion | n.a. |
| PDE_B18080 B | Batch | 50' |
| PDE_B18080 C | Batch | n.a. |

During the other phases of the process, all three bioreactors followed the standard conditions set in the previous run and reported in Table 12.

**Table 12: Experimental schedule**

| **Day** | **Step description** |
|---|---|
| **1** | -Inoculum at day 1, 5000 cells/cm². |
| **3** | -Sample from bioreactor to monitor pH and glu/lat. |
| | -Medium change pre- transfection in perfusion (650 mL). |
| | -Pei -mediated transfection according to protocol described in Example 1.3 |
| **4** | -Sample from bioreactor to monitor pH and glu/lat, and to evaluate contaminants. |
| | -Medium change post transfection. |
| | -Sample from bioreactor to evaluate contaminants. |
| | -Start harvest in perfusion |
| **5** | -Sample from bioreactor to monitor pH and glu/lat. |
| | -Sample from out bottle to test p24. |
| **6** | -Sample from bioreactor to monitor pH and glu/lat. |
| | -Sample from out bottle to test p24. |
| | -Harvest of total volume of supernatant-Sample from total pool to test physical and infectious viral titer. |

Total pool of three bioreactors were tested for physical and infectious viral titer, HCP, plasmid and total residual DNA.

The results are reported in Table 13 and Table 14:

**Table 13: Productivity data**

| **Process ID** | **Physical viral titer** | | | **Infectious viral titer** | | | **Infectivity** |
|---|---|---|---|---|---|---|---|
| | **ngP24/mL** | **tot ngP24** | **ngP24/cm²** | **TU/mL** | **Total TU** | **TU/cm2** | **TU/ngP24** |
| **PDE_B18080 A** | 158 | 2.05E+05 | 38.00 | 1.6E+07 | 2.08E+10 | 3.92E+06 | 1.01E+05 |
| **PDE_B18080 B** | 282 | 3.67E+05 | 69.00 | 2.0E+07 | 2.60E+10 | 4.91E+06 | 7.09E+04 |
| **PDE_B18080 C** | 217 | 2.82E+05 | 53.00 | 2.0E+07 | 2.60E+10 | 4.91E+06 | 9.22E+04 |

**Table 14: Residuals data**

| **Process ID** | **HCP (ng/mL)** | **Total DNA (ng/mL)** |
|---|---|---|
| **PDE_B18080 A** | 1882 | 997 |
| **PDE_B18080 B** | 2885 | 1597 |
| **PDE_B18080 C** | 2494 | 1262 |

As results show, it was surprisingly found that the productivity of LV vectors was lower in the bioreactor A, performed with medium change post transfection in perfusion mode (PDE_B18080 A), than the run performed with medium change post transfection performed in batch (PDE_B18080 C). The productivity of run PDE_B18080B, with medium change post transfection performed in batch mode with holding time, was also higher than that obtained in the run performed with medium change post transfection performed in perfusion (PDE_B18080 A), and even higher than the run PDE_B18080 C, while the infectious viral titer of run PDE_B18080 B is the same of the run PDE_B18080 C. The amount of contaminants, HCP, plasmid and total DNA is equivalent between the two runs PDE_B18080 B and PDE_B18080 C. In view of the scaling up to large scale in iCellis 500, medium change in batch with holding time in iCellis nano is more consistent with run performed in large scale iCellis 500 (since 50' is the time needed for empty and filling the bioreactor). Therefore, it is more representative of the performance of the run in large scale.

### EXAMPLE 2 - LENTIVIRAL VECTOR PRODUCTION IN iCELLis NANO 1,06 m²

### EXAMPLE 2.1 - MATERIALS AND METHODS

### PACKAGING CELLS AND CELL CULTURE MEDIUM

The production of the LVV vectors described in this Example 2.1 has been performed with the HEK 293T cell line. HEK 293T cells were thawed and expanded in IMDM 10% FBS, 2% Glutamax.

### BIOREACTOR

Except for the first experiment of example2.2, in which one run has been performed in iCellis Nano having a surface area of 0.53m² bioreactor, all the other runs described in examples 2.2 and 2.3 have been performed with iCellis Nano having a surface area of 1.06m² bioreactor. Characteristics of the two kind of support are reported in Table 15.

**Table 15: iCELLis nano characteristics**

| **Bioreactor Size (m²)** | **FB height (cm)** | **Compaction** | **Carrier Density (g/L)** | **No. of carriers** |
|---|---|---|---|---|
| 0.53 | 2 | 1 | 96 | 473 |
| 1.06 | 4 | 1 | 96 | 946 |

### PLASMIDS

The production of the LVV vectors in iCELLis nano in this example 2 a multi-plasmid DNA system used is a third generation LVVs packaging system including 4 separate plasmids: one plasmid carrying gag-pol genes (pGag/Pol), one plasmid encoding rev (pREV), one plasmid carrying VSV-g gene (pENV-VSV-G)), and one transfer vector plasmid carrying the gene encoding GFP protein (pTransfer-GFP).

### ANALYTICAL METHODS

The following analytical methods has been applied to evaluate process performance:
- Physical viral titer: this analytical method is an ELISA to quantify p24 HIV protein, which can be used to evaluate the concentration of physical particles.
- Infectious Viral Titer: this analytical method is based on transduction of reference CEM A3.01 cell line. The expression of the GFP protein is evaluated by FACS analysis.
- Residual Host Cell Proteins: this analytical method is an ELISA to quantify residual proteins from HEK-293T packaging cell line in LVV samples.
- Residual Total DNA: this analytical method is based on a fluorescent dye that stains and allows quantification of residual DNA.

### SUMMARY OF THE MANUFACTURING PROCESS

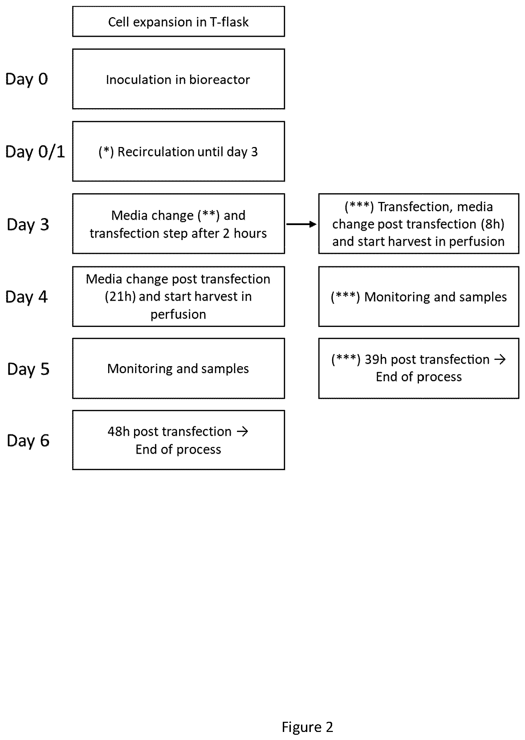

A flow chart of the processes applied in experiments disclosed in Example 2 is reported in Figure 2. Briefly, for each experiment cells have been expanded in T-flasks in IMDM w/o phenol red and containing 10% FBS and 2% Glutamax. After split, cells have been inoculated in the bioreactor iCELLis nano (5000 cells/cm²). After expansion phase in which pH, DO%, glucose and lactate concentration were monitored, the culture medium was changed (in perfusion or through recirculation) with fresh complete medium and transfection step was performed at day 3. After 21 hours a media change in batch was performed in order to remove all residuals of plasmid DNA and PEI Pro. Harvest was performed in perfusion mode. Lentiviral vector supernatant has been collected 48 hours after media change post transfection. From run PDE_B18030 on, media change post transfection and harvest were anticipate at day 3 and day 5 respectively, to collect a vector supernatant with higher infectivity. Lentiviral vector's samples during harvest in perfusion have been filtered 0.45µm and stored at <-65°C for testing.

### TRANSFECTION STEP

In all the experiments reported in this example 2, production of LVV vector was performed using Polyethylenimine (PEI)-mediated transfection method. The optimization study was performed using the amount of µg of plasmid/mL previously tested in Example 1. Till the run PDE_B18187 DNA/cell ratio was maintained, doubling the volume of transfection mix and the quantity of DNA and PEl in 1.06m2 bioreactor (1924µg total DNA). Then the amount of DNA/cell was halved, using the same volume of transfection mix and DNA/mL ratio of 0.53m2 iCELLis nano (962 µg total DNA). The amount of PEI used has been set to maintain a ratio of 1:1 between PEI and DNA.

Protocol for transfection at day 3:
1. Perform medium change in perfusion (only if provided) with pre-warmed complete medium 1 hour before transfection.
2. Prepare a total DNA mix for the bioreactors, diluting the correct amount of DNA in IMDM free
3. Dilute the correct amount of PEI in IMDM free
4. Transfer the diluted PEI in the tube containing the diluted DNA and mix.
5. Incubate the final mix for according to the manufacturer instructions .
6. Remove the correct volume from bioreactor, transfer the mix into the bioreactor.

### EXAMPLE 2.2 - MANUFACTURING IN ICELLIS NANO 1.06M² VS ICELLIS NANO 0.53M²

The goal of this first group of experiments was to test:
- Bioreactor with 1.06m² area;
- Recirculation during cell culture;
- Reduction of harvest volume;

In all these experiments, 5000 cells/cm² were inoculated in iCELLis nano at day 0. After an expansion phase in flask T225, 26.5 x10⁶ cells (in 0.53m² bioreactor) and 53 x10⁶ cells (in 1.06m² bioreactor) were inoculated in the bioreactor. Control Petri dishes have been seeded in parallel at 5.50x10⁵cells/petri in order to monitor the transfection phase.

Sampling have been done from bioreactor during the process in order to measuring pH, lactate and glucose concentration. In the first two runs, at day 3, three carriers were taken from each bioreactor and lysate; a nuclei count was performed in order to estimate the cell density for cm².

Recirculation mode during cell culture for expansion has been evaluated. For all experiments, PEI-mediated transfection was performed according to the protocol described in Example 2.1. The transfection mix for the 0.53m² iCELLis nano was prepared using 962µg of total DNA (corresponding to 181ng/cm² of surface area) and 962µg PEI in a total volume of 100ml. In 1.06m² iCELLis nano, volume of transfection mix and quantity of DNA and PEI have been doubled. 21 hours after transfection, the exhausted culture medium was removed and, after a holding time of 50 minutes, replaced with fresh medium for production. Harvests in perfusion was performed till 48h after media change post transfection

**Table 16: Experimental schedule**

| **Process ID** | **Area (m²)** | **Media change pre transf.** | **Recirculation** | **Transfection mix** |
|---|---|---|---|---|
| | | | | **DNA (µg)** |
| **PDE_B18172 Bio1** | 0.53 | Yes | no | 962 |
| **PDE_B18172 Bio2** | 1.06 | Yes | (1300mL) | 1924 |
| **PDE_B18172 Bio3** | 1.06 | No | (1950mL) | 1924 |
| **PDE_B18187 Bio1** | 1.06 | No | (1300mL) | 1924 |
| **PDE_B18187 Bio2** | 1.06 | No | (1300mL) | 1924 |
| **PDE_B18187 Bio3** | 1.06 | No | (1300mL) | 1924 |
| **PDE_B19002 Bio1** | 1.06 | No | (1300mL) | 1924 |
| **PDE_B19002 Bio2** | 1.06 | No | (1300mL) | 1924 |
| **PDE_B19002 Bio3** | 1.06 | No | (1300mL) | 1924 |

The number of cells/cm² and the amount of lactate produced (mg/mL) have been evaluated to monitor growth rate and are reported in Table 17.

**Table 17: Cell densisty (cells/cm²) and lactate produced (mg/mL)**

| | **Day 1** | **Day 3** | | **Day 4** | **Day 5** | | **Day 6** | | |
|---|---|---|---|---|---|---|---|---|---|
| **Process ID** | **Lac in** | **Lac in** | **cells/cm²** | **Lac in** | **Lac in** | **Lac out** | **Lac in** | **Lac out** | **cells/cm²** |
| **PDE_B18172 Bio1** | 0.265/ 0.261 | 0.850/ 0.842 | 0.88E+05 | 1.52/ 1.52 | 1.27/ 1.23 | n.a. | 1.48/ 1.53 | 1.03/ 1.07 | 3.1E+05 |
| **PDE_B18172 Bio2** | 0.409/ 0.411 | 0.964/ 0.945 | 1.39E+05 | 1.95/ 2.18 | 0.952/ 0.934 | n.a. | 1.18/ 1.13 | 0.919/ 0.899 | 3.1E+05 |
| **PDE_B18172 Bio3** | 0.362/ 0.368 | 0.684/ 0.663 | 1.06E+05 | 2.00/ 2.10 | 0.797/ 0.815 | n.a. | 0.937/ 0.917 | 0.788/ 0.767 | 2.3E+05 |
| **PDE_B18187 Bio1** | 0.422/ 0.417 | 0.914/ 0.901 | 1.40E+05 | 2.36/ 2.35 | 1.13/ 1.11 | n.a. | 1.20/ 1.21 | 1.02/ 1.00 | n.a. |
| **PDE_B18187 Bio2** | 0.261/ 0.264 | 0.853/ 0.871 | 1.20E+05 | 2.28/ 2.29 | 1.12/ 1.13 | n.a. | 1.25/ 1.25 | 1.01/ 1.00 | n.a. |
| **PDE_B18187 Bio3** | 0.429/ 0.435 | 0.833/ 0.848 | 0.93E+05 | 2.25/ 2.34 | 1.13/ 1.14 | n.a. | 1.19/ 1.20 | 0.973/ 1.01 | n.a. |
| **PDE_B19002 Bio1** | n.a. | 0.838 | n.a. | 1.28 | 0.880 | 0.695 | 1.04 | 0.808 | 7.3E+05 |
| **PDE_B19002 Bio2** | n.a. | 0.821 | n.a. | 1.19 | 1.24 | 0.892 | 1.46 | 1.10 | 4.8 E+05 |
| **PDE_B19002 Bio3** | n.a. | 0.853 | n.a. | 1.21 | 1.03 | 0.780 | 1.14 | 0.942 | 4.2E+05 |

The results of viral titer in terms of P24 and TU are summarized in Table 18.

**Table 18: Productivity data**

| | **Physical viral titer** | | | **Infectious viral titer** | | | **Infectivity** |
|---|---|---|---|---|---|---|---|
| **Process ID** | **ngP24/mL** | **tot ngP24** | **ngP24/cm²** | **TU/mL** | **Total TU** | **TU/cm2** | **TU/ngP24** |
| **PDE_B18172 petri** | 440 | n.a. | n.a. | 1.6E+07 | n.a. | n.a. | 3.6E+04 |
| **PDE_B18172 Bio1** | 151 | 1.96E+05 | 37 | 7.0E+06 | 9.1E+09 | 1.7E+06 | 4.6E+04 |
| **PDE_B18172 Bio2** | 253 | 6.70E+05 | 62 | 1.2E+07 | 3.1E+10 | 2.9E+06 | 4.7E+04 |
| **PDE_B18172 Bio3** | 281 | 7.45E+05 | 69 | 1.2E+07 | 3.1E+10 | 2.9E+06 | 4.3E+04 |
| **PDE_B18187 Bio1** | 182 | 4.82E+05 | 45 | 1.0E+07 | 2.6E+10 | 2.5E+06 | 5.5E+04 |
| **PDE_B18187 Bio2** | 227 | 6.02E+05 | 56 | 1.5E+07 | 3.9E+10 | 3.7E+06 | 6.6E+04 |
| **PDE_B18187 Bio3** | 221 | 2.41E+05 | 45 | 1.2E+07 | 2.6E+10 | 2.4E+06 | 5.4E+04 |
| **PDE_B19002 Bio1** | 226 | 5.88E+05 | 55 | 9.4E+06 | 2.4E+10 | 2.3E+06 | 4.2E+04 |
| **PDE_B19002 Bio2** | 347 | 6.21E+05 | 70 | 1.5E+07 | 3.2E+10 | 3.0E+06 | 4.3E+04 |
| **PDE_B19002 Bio3** | 347 | 8.11E+05 | 70 | 1.5E+07 | 3.2E+10 | 2.3E+06 | 4.3E+04 |

The amounts of HCP and total DNA of the pool harvest are reported in Table 19.

**Table 19: HCP and total DNA (ng/mL) amounts**

| | **HCP** | **Tot DNA** |
|---|---|---|
| **Process ID** | **Pool harvest** | **Pool harvest** |
| **PDE_B18172 Bio1** | 1028 | 514 |
| **PDE_B18172 Bio2** | 2760 | 1766 |
| **PDE_B18172 Bio3** | 2831 | 1532 |
| **PDE_B18187 Bio1** | 1757 | 1144 |
| **PDE_B18187 Bio2** | 1981 | 1319 |
| **PDE_B18187 Bio3** | 2026 | 1337 |
| **PDE_B19002 Bio1** | 2151 | 1801 |
| **PDE_B19002 Bio2** | 2263 | 1479 |
| **PDE_B19002 Bio3** | 1840 | 1176 |

The results demonstrate that the infectious viral titer of vector produced in 1.06m² iCELLis bioreactor is higher than that produced in 0.53m² iCELLis nano. Recirculation mode doesn't cause any worsening to cell productivity of LVV vectors and replaces media change.

### EXAMPLE 2.3 - MANUFACTURING IN iCELLIS NANO 1.06m²

In this example 2.3 the conditions set in the previous runs (example 2.2) were maintained:
- 1.06m² iCELLis bioreactor;
- No media change pre transfection;
- Recirculation during cell culture;
- Harvesting in perfusion.

Transfection step in a bioreactor having 1.06m2 surface area and media change post transfection were investigated. In run PDE_B18197 different ratio of DNA/n° of cells and DNA/mL have been tested. In Bio 1 same transfection condition used in Example 2.2 were applied (i.e.: 1924µg total DNA, i.e: 181ng/cm2 of surface area, 1924µg PEI, in transfection mix volume of 200 ml). In Bio2, halved volume of transfection mix (100ml) has been used compared to the previous experiments with 1.06m² bioreactor. So the DNA/n° of cells ratio has been maintained, but the concentration of DNA and PEI was twice as much as the total volume. In Bio3 the same volume (100 ml) and amount of DNA (962µg, i.e: 91ng/cm2 of surface area) and PEI (962µg) used for 0.53m² iCELLis nano has been tested. In this way DNA/mL ratio has been maintained, but the amount of DNA and PEI was half compared to the number of cells.

The different conditions used are summarizes in Table 20.

**Table 20: Amount of plasmid DNA and PEI**

| **Plasmid** | **µg /Bioreactor** | | |
|---|---|---|---|
| | **Bio1*** | **Bio2**** | **Bio3**** |
| **Total DNA** | **1924** | **1924** | **962** |
| **Total PEI-PRO (1µg/µl)** | **1924** | **1924** | **962** |

| | | | |
|---|---|---|---|
| *In 200mL. **In 100mL. | | | |

In run PDE_B19013 conditions of PDE_B18197 Bio3 have been reproduced, but different timing for transfection step and following media change has been tested. In Bio1 previous conditions were maintained (transfection at 11.30 on Monday and media change post transfection on Tuesday morning); in Bio2 both transfection step and media change have been performed on Monday, respectively at 9.30 and 17.30. Bio3 has been carried out as Bio2, but with a delay of 2 hours from Monday on. In all 3 bioreactors harvest in perfusion has started after media change post transfection.

Brefly, 5000 cells/cm² were inoculated in iCELLis nano at day 0. Media recirculation has been done during cell culture for transfection. Then, transfection step and media change post transfection in batch have been performed as previously described. Harvest in perfusion has started after media change post transfection.

Sampling have been done from day 3 to day 6 (morning and afternoon) in order to monitor cell growth.

Experimental conditions are summarized in Table 21.

**Table 21: Experimental schedule**

| **Process ID** | **Transfection** | **Media change** | **Harvest in perfusion** |
|---|---|---|---|
| **PDE_B18197 Bio1** | 21h | Tuesday | 48h |
| **PDE_B18197 Bio2** | 21h | Tuesday | 48h |
| **PDE_B18197 Bio3** | 21h | Tuesday | 48h |
| **PDE_B19013 Bio1** | 21h | Tuesday | 48h |
| **PDE_B19013 Bio2** | 8h | Monday | 63h |
| **PDE_B19013 Bio3** | 8h | Monday | 61h |

The number of cells/cm² and the amount of lactate produced (mg/mL) have been evaluated to monitor growth rate and are reported in Table 22.

**Table 22: Cell densisty (cells/cm²) and lactate produced (mg/mL)**

| | | **Day 3** | | **Day 4** | | **Day 5** | | **Day 6** | | |
|---|---|---|---|---|---|---|---|---|---|---|
| **Process ID** | | **Lac in** | **cells/c m²** | **Lac in** | **Lac out** | **Lac in** | **Lac out** | **Lac in** | **Lac out** | **cells/c m²** |
| **PDE_B18197 Bio1** | | 0.928/ 0.916 | 1.44E +05 | 2.21/ 2.33 | n.a. | 0.811/ 0.821 | 0.674/ 0.694 | 0.962/ 0.983 | 0.786/ 0.800 | 5.12E +05 |
| **PDE_B18197 Bio2** | | 0.944/ 0.942 | 1.95E +05 | 2.47/ 2.48 | n.a. | 1.09/ 1.13 | 0.833/ 0.872 | 1.28/ 1.29 | 1.03/ 1.04 | 2.26E +05 |
| **PDE_B18197 Bio3** | | 0.901/ 0.900 | 1.64E +05 | 2.20/ 2.19 | n.a. | 0.943/ 0.951 | 0.702/ 0.708 | 1.08/ 1.09 | 0.858/ 0.866 | 0.50E +05 |
| **PDE_B19013 Bio1** | Morning | 0.687 | n.a. | 2.01 | n.a. | 0.964 | 0.722 | 1.22 | 0.843 | n.a. |
| | Afternoon | n.a. | n.a. | 0.609 | 0.464 | 1.03 | 0.739 | n.a. | n.a. | n.a. |
| **PDE_B19013 Bio2** | Morning | 0.717 | n.a. | 0.835 | 0.648 | 1.27 | 0.928 | 1.54 | 1.08 | n.a. |
| | Afternoon | 1.25 | n.a. | 1.04 | 0.737 | 1.31 | 0.936 | n.a. | n.a. | n.a. |
| **PDE_B19013 Bio3** | Morning | 0.660 | n.a. | 0.716 | 0.544 | 1.02 | 0.757 | 1.25 | 0.869 | n.a. |
| | Afternoon | 1.24 | n.a. | 0.842 | 0.608 | 1.03 | 0.767 | n.a. | n.a. | n.a. |

The results of physical and infectious viral titer and infectivity of pool harvest are summarized in Table 23.

**Table 23: Productivity data**

| **Process ID** | | **Physical viral titer** | | | **Infectious viral titer** | | | **Infectivity** |
|---|---|---|---|---|---|---|---|---|
| | | **ngP24/ mL** | **tot ngP24** | **ngP24/ cm²** | **TU/mL** | **Total TU** | **TU/cm2** | **TU/ngP24** |
| **PDE_B18197 Bio1** | | 231 | 6.01E+05 | 58 | 9.0E+06 | 2.3E+10 | 2.2E+06 | 3.9E+04 |
| **PDE_B18197 Bio2** | | 198 | 5.15E+05 | 50 | 7.0E+06 | 1.8E+10 | 1.7E+06 | 3.5E+04 |
| **PDE_B18197 Bio3** | | 231 | 6.01E+05 | 58 | 1.1E+07 | 2.9E+10 | 2.7E+06 | 4.8E+04 |
| **PDE_B19013 Bio1** | * | 229 | 3.78E+05 | 36 | 7.8E+06 | 1.3E+10 | 1.2E+06 | 3.4E+04 |
| | ** | 267 | 5.74E+05 | 65 | 1.0E+07 | 2.2E+10 | 2.0E+06 | 3.7E+04 |
| **PDE_B19013 Bio2** | * | 186 | 3.63E+05 | 34 | 1.4E+07 | 2.7E+10 | 2.6E+06 | 7.5E+04 |
| | ** | 194 | 5.02E+05 | 47 | 9.9E+06 | 2.6E+10 | 2.4E+06 | 5.1E+04 |
| **PDE_B19013 Bio3** | * | 172 | 3.35E+05 | 32 | 1.1E+07 | 2.2E+10 | 2.0E+06 | 6.5E+04 |
| | ** | 198 | 5.00E+05 | 47 | 9.4E+06 | 2.4E+10 | 2.2E+06 | 4.7E+04 |

| | | | | | | | | |
|---|---|---|---|---|---|---|---|---|
| *: Wednesday **:Thursday | | | | | | | | |

The amounts of HCP and total DNA of the pool harvest are reported in Table 24.

**Table 24: HCP and total DNA (ng/mL) amounts**

| **Process ID** | | **HCP** | **Tot DNA** |
|---|---|---|---|
| **PDE_B18197 Bio1** | | 2116 | 1192 |
| **PDE_B18197 Bio2** | | 1794 | 864 |
| **PDE_B18197 Bio3** | | 1870 | 1058 |
| **PDE_B19013 Bio1** | * | <866 | 1727 |
| | ** | 1285 | 1370 |
| **PDE_B19013 Bio2** | * | <LLOQ (624 ng/ml) | neg. |
| | ** | <LLOQ (624 ng/ml) | neg. |
| **PDE_B19013 Bio3** | * | <LLOQ (624 ng/ml) | 545 |
| | ** | <LLOQ (624 ng/ml) | 959 |

The results show that the production of LVV vector in terms of P24 in experiment PDE_B18197 Bio3 is the same of Bio1, and higher than Bio 2 demonstrating that, surprisingly, it was possible to obtain the same or even higher productivity using half of the total amount of DNA. Moreover, this condition improves the infectious viral titer, bringing to an increase of infectivity. Reduction of time of transfection from 21h to 8h in run PDE_B19013 does not reduce much the physical viral titer, but increases the infectivity. In particular, the results show that the infectivity of Bio2 and Bio3 on Wednesday is higher than in the other run, in which the harvest had been performed till Thursday. The results of HCP and Total DNA of run PDE_B19013 Bio2 and Bio3 are lower than in the previous run, demonstrating that supernatant has a lower presence of impurities.

### EXAMPLE 2.4 - TRANSFECTION AND HARVESTING IN THREE DAYS

In order to confirm the results obtained, the conditions previously tested in example 2.3 and chosen as the best have been performed again in run described in the Table 25.

**Table 25: Experimental schedule**

| **Process ID** | **Transfection mix** | **Transfection** | **Media change** | **Harvest in perfusion** |
|---|---|---|---|---|
| | **DNA (µg)** | | | |
| **PDE_B19030 Bio1** | 962 | 8h | Monday | 39h |
| **PDE_B19030 Bio2** | 962 | 21h | Tuesday | 48h |
| **PDE_B19030 Bio3** | 962 | 8h | Monday | 39h |
| **PDE_B19041 Bio2** | 962 | 8h | Monday | 39h |
| **PDE_B19041 Bio3** | 962 | 8h | Monday | 39h |
| **PDE_B19048 Bio1** | 962 | 8h | Monday | 39h |
| **PDE_B19048 Bio2** | 962 | 8h | Monday | 39h |
| **PDE_B19048 Bio3** | 962 | 8h | Monday | 39h |
| **PDE_B19054 Bio1** | 962 | 8h | Monday | 39h |
| **PDE_B19054 Bio2** | 962 | 8h | Monday | 39h |
| **PDE_B19054 Bio3** | 962 | 8h | Monday | 39h |

As in the previous run, 5000 cells/cm² were inoculated in iCELLis nano at day 0. Media recirculation has been performed up to day 3 with 1300mL of fresh medium. PEl-mediated transfection has been performed at 10.00 am and media change post transfection in batch have been done 8 hours later, both on day3. Harvest in perfusion has started after media change post transfection. Sampling have been done from day 3 to day 6 in order to monitor cell growth. The amount of lactate produced (mg/mL) have been evaluated to monitor growth rate and are reported in Table 26.

**Table 26: Lactate produced (mg/mL)**

| | **Day 3** | | **Day 4** | | **Day 5** | | **Day 6** | |
|---|---|---|---|---|---|---|---|---|
| **Process ID** | **Lac in** | | **Lac in** | **Lac out** | **Lac in** | **Lac out** | **Lac in** | **Lac out** |
| | **Morning** | **Afternoon** | | | | | | |
| **PDE_B19030 Bio1** | 0.809 | 1.37 | 0.716 | 0.544 | 1.04 | 0.736 | n.a. | n.a. |
| **PDE_B19030 Bio2** | 0.855 | n.a. | 2.30 | n.a. | 1.13 | 0.795 | 1.49 | 1.03 |
| **PDE_B19030 Bio3** | 0.834 | 1.49 | 0.832 | 0.600 | 1.13 | 0.808 | n.a. | n.a. |
| **PDE_B19041 Bio2** | 0.559 | 1.24 | 0.874 | 0.647 | 1.02 | 0.799 | | |
| **PDE_B19041 Bio3** | 0.613 | 1.31 | 0.857 | 0.637 | 1.03 | 0.769 | | |
| **PDE_B19048 Bio1** | 0.722 | 1.17 | 0.656 | 0.522 | 0.900 | 0.665 | | |
| **PDE_B19048 Bio2** | 0.787 | 1.30 | 0.891 | 0.657 | 1.22 | 0.915 | | |
| **PDE_B19048 Bio3** | 0.720 | 1.23 | 0.676 | 0.542 | 0.860 | 0.658 | | |
| **PDE_B19054 Bio1** | 0.671 | 1.25 | 0.713 | 0.576 | 0.880 | 0.702 | | |
| **PDE_B19054 Bio2** | 0.790 | 1.34 | 0.866 | 0.660 | 1.10 | 0.887 | | |
| **PDE_B19054 Bio3** | 0.677 | 1.15 | 0.782 | 0.583 | 1.02 | 0.860 | | |

The results of physical and infectious viral titer and infectivity of final pool harvest are reported in Table 27.

**Table 27: Productivity data**

| **Process ID** | **Physical viral titer** | | | **Infectious viral titer** | | | **Infectivity** |
|---|---|---|---|---|---|---|---|
| | **ngP24/ mL** | **tot ngP24** | **ngP24/ cm²** | **TU/mL** | **Total TU** | **TU/cm²** | **TU/ngP24** |
| **PDE_B19030 Bio1** | 302 | 0.59E+06 | 56 | 1.7E+07 | 3.2E+10 | 3.1E+06 | 5.5E+04 |
| **PDE_B19030 Bio2*** | 329* | 0.60E+06* | 68* | 1.1E+07* | 2.5E+10* | 2.4E+06* | 3.5E+04* |
| **PDE_B19030 Bio3** | 248 | 0.48E+06 | 46 | 1.7E+07 | 3.4E+10 | 3.2E+06 | 7.0E+04 |
| **PDE_B19041 Bio2** | 347 | 0.68E+06 | 64 | 3.6E+07 | 4.7E+10 | 8.8E+06 | 6.0E+04 |
| **PDE_B19041 Bio3** | 317 | 0.62E+06 | 58 | 2.1E+07 | 4.1E+10 | 3.9E+06 | 5.4E+04 |
| **PDE_B19048 Bio1** | 257 | 0.50E+06 | 47 | 1.7E+07 | 3.4E+10 | 3.2E+06 | 5.1E+04 |
| **PDE_B19048 Bio2** | 389 | 0.76E+06 | 72 | 1.3E+07 | 2.6E+10 | 2.4E+06 | 5.2E+04 |
| **PDE_B19048 Bio3** | 391 | 0.76E+06 | 72 | 2.0E+07 | 4.0E+10 | 3.7E+06 | 4.1E+04 |
| **PDE_B19054 Bio1** | 402 | 0.78E+06 | 74 | 1.6E+07 | 3.1E+10 | 2.9E+06 | 6.7E+04 |
| **PDE_B19054 Bio2** | 422 | 0.82E+06 | 78 | 2.7E+07 | 5.3E+10 | 5.0E+06 | 7.5E+04 |
| **PDE_B19054 Bio3** | 283 | 0.55E+06 | 52 | 3.3E+07 | 6.4E+10 | 6.1E+06 | 5.2E+04 |

| | | | | | | | |
|---|---|---|---|---|---|---|---|
| * Physical titer, viral titer and infectivity on day 6 instead of day 5.The amounts of HCP and total DNA of the pool harvest are reported in Table 28. | | | | | | | |

**Table 28: HCP and total DNA (ng/mL) amounts**

| **Process ID** | **HCP** | **Tot DNA** |
|---|---|---|
| **PDE_B19030 Bio1** | 989 | <LLOQ |
| **PDE_B19030 Bio2** | 1543 | 517 |
| **PDE_B19030 Bio3** | 769 | < LLOQ (<0.6 µg/mL) |
| **PDE_B19041 Bio2** | 578 | neg. |
| **PDE_B19041 Bio3** | 719 | neg. |
| **PDE_B19048 Bio1** | 696 | < LOQ (624 ng/ml) |
| **PDE_B19048 Bio2** | 1159 | < LOQ (624 ng/ml) |
| **PDE_B19048 Bio3** | 379 | < LOQ (624 ng/ml) |
| **PDE_B19054 Bio1** | 726 | <LLOQ(<624ng/mL) |
| **PDE_B19054 Bio2** | 784 | <LLOQ(<624ng/mL) |
| **PDE_B19054 Bio3** | <LLOQ (<480) | <LLOQ(<624ng/mL) |

As the results show, infectious viral titer and infectivity are higher when media change post transfection and final harvest are anticipate by one day i.e.: when transfection and harvesting are performed in three days. Moreover, pool harvests obtained performing transfection and harvesting in a total of three days result to have less contaminants with total DNA being undetectable or below the analytical method limit.

### EXAMPLE 3 - MANUFACTURING IN iCellis 500

### EXAMPLE 3.1 - MANUFACTURING IN iCellis 500 HAVING SURFACE AREA 66m²

Two parallel vector productions in iCellis500 having a surface are of 66 m² and iCellis nano having a surface area of 0,53m² have been performed as summarized in Table 29. The processes were performed using 293-T cell line and the third generation multi plasmid DNA system used in examples 1 and 2.

**Table 29: Experimental schedule of the production runs**

| **Days** | **Process Step for iCELLis500 vector production (PDE_BI8116)** | **Process Step for iCELLis nano vector production (PDE_S18117)** |
|---|---|---|
| Day 0 | Inoculum seed cells in the bioreactor at 0.5x10⁴/cm² | |
| | 0.5x10⁴/cm² x 66m²= 3.3 x10⁹ | 0.5x10⁴/cm² x 0.053m²=26.5 x10⁶ |
| Day 3 | • pre-transfection medium change in perfusion | • pre-transfection medium change in perfusion |
| | • Addition of transfection mix containing multi-plasmid DNA (181ng/cm²) and PEI (ratio 1:1) | Addition of transfection mix **containing multi-plasmid DNA (181ng/cm²) and PEI (ratio 1:1)** |
| Day 4 | • Post-transfection medium change in batch | • Post-transfection medium change in batch (50' of holding time) |
| | • start harvest in perfusion | • start harvest in perfusion |
| Day 6 | • Stop perfusion and empty the vector inside the bioreactor | • Stop perfusion and empty the vector inside the bioreactor: |

The comparability between iCellis500 and iCellis nano of post clarification pre benzonase sample are reported in Table 30.

**Table 30: Comparability between iCellis500 and iCellis nano**

| | iCellis500 | iCellisnano |
|---|---|---|
| Physica Particles (ng/cm²) | 33 | 39 |
| Viral Titer (TU/cm²) | 3.2E+06 | 2.6E+06 |
| Infectivity (TU/ng p24) | 9.1E+04 | 6.6E+04 |
| Host Cell Protein content (HCP) (ng/cm²) | 516 | 357 |
| Total DNA content(ng/cm²) | 302 | 230 |
| BSA(µg/cm²) | 414 | 388 |

The results show that by upscaling the process to iCellis 500 productivity is maintained and infectious viral titer and infectivity result to be improved in respect to data obtained with iCellis nano. The impurity profile of the 2 runs is comparable and acceptable.

### EXAMPLE 3.2 - MANUFACTURING IN iCellis 500 HAVING SURFACE AREA 133 m²

Two parallel vector productions in iCellis500 having a surface are of 133 m² and iCellis nano having a surface area of 1,06 m² have been performed as summarized in Table 31. The processes were performed using 293-T cell line and the third generation multi plasmid DNA system used in examples 1 and 2.

**Table 31: Experimental schedule of the production runs**

| **Days** | **Process Step for iCELLis500 vector production (PDE_B19130)** | **Process Step for iCELLis nano vector production (PDE_B19129)** |
|---|---|---|
| Day 0 | Inoculum: seed cells in the bioreactor 133m² at 0.5x10⁴/cm² | |
| | 0.5x10⁴/cm² x 133m²= 6.65 x10⁹ | 0.5x10⁴/cm² x 0.106m²=53 x10⁶ |
| | Start recirculation with 176.1 L of complete medium | Start recirculation 1.3 L of complete medium |
| Day 3 | Addition of transfection mix **containing multi-plasmid DNA (91ng/cm²) and PEI (ratio 1:1)** | Addition of transfection mix **containing multi-plasmid DNA (91ng/cm²) and PEI (ratio 1:1)** |
| | Post-transfection medium change in batch of complete medium | Post-transfection medium change in batch (50' of holding time) |
| | Start harvest in perfusion | Start harvest in perfusion |
| Day 5 | Stop perfusion and empty the vector inside the bioreactor | Stop perfusion and empty the vector inside the bioreactor |

The comparability between iCellis500 and iCellis nano of pre clarification sample are reported in Table 32.

**Table 32: Comparability between iCellis500 and iCellis nano**

| | iCellis500 | iCellisnano* |
|---|---|---|
| Physical Particles(ng/cm²) | 66 | 61 |
| Viral Titer (TU/cm²) | 7.7E+06 | 3.7E+06 |
| Infectivity (TU/ngp24) | 1.2E+05 | 6.0E+04 |
| Host Cell Protein content (HCP) (ng/cm²) | 140 | 223 |
| Total DNA content (ng/cm²) | < LLOQ | < LLOQ |

The results show that by upscaling the process to iCellis 500 productivity is maintained and infectious viral titer and infectivity result to be improved (doubled infectious viral titer and infectivity) in respect to data obtained with iCellis nano. The impurity profile of the bulk productions obtained in the 2 runs is comparable and the total DNA content of the production at large scale as well as at small scale results to be below the detection limit of the analytical method used, such results are obtained on samples collected before the treatment with endonuclease.

### EXAMPLE 4 - MINIMUM TOTAL DNA QUANTITY

The goal of this experiment is the evaluation of the productivity of the process obtained by performing transfection with DNA at total quantity of 45ng/cm² and 60ng/cm².

### PACKAGING CELLS AND CELL CULTURE MEDIUM

The production of the LVV vectors described in this Example 4 has been performed with the HEK 293T cell line. HEK 293T cells were thawed and expanded in IMDM 10% FBS, 2% Glutamax.

### BIOREACTOR

The experiment was performed with iCellis Nano having a surface area of 1.06m² bioreactor.

**Table 33: iCELLis nano characteristics**

| **Bioreactor Size (m²)** | **FB height (cm)** | **Compaction** | **Carrier Density (g/L)** | **No. of carriers** |
|---|---|---|---|---|
| 1.06 | 4 | 1 | 96 | 946 |

### PLASMIDS

For the production of the LVV vectors in iCELLis nano in this example 4 the multi-plasmid DNA system used is a third generation LVVs packaging system including 4 separate plasmids: one plasmid carrying gag-pol genes (pGag/Pol), one plasmid encoding rev (pREV), one plasmid carrying VSV-g gene (pENV-VSV-G)), and one transfer vector plasmid carrying the gene encoding GFP protein (pTransfer-GFP).

### ANALYTICAL METHODS

The following analytical methods has been applied to evaluate process performance:
- Physical viral titer: this analytical method is an ELISA to quantify p24 HIV protein, which can be used to evaluate the concentration of physical particles.
- Infectious Viral Titer: this analytical method is based on transduction of reference CEM A3.01 cell line. The expression of the GFP protein is evaluated by FACS analysis.

### SUMMARY OF THE MANUFACTURING PROCESS

The process performed in this example 4 is summarized in table 34.

**Table 34: process steps**

| **Days** | **Process Steps** |
|---|---|
| Day 0 | Inoculum: seed cells in the bioreactor at 0.5x10⁴/cm²: i.e. total cells 0.5x10⁴/cm² x 0.106m²=53 x10⁶ |
| | Start recirculation 1.3 L of complete medium |
| Day 3 | Addition of transfection mix containing multi-plasmid DNA and PEI (ratio 1:1) |
| | Post-transfection medium change in batch (50' of holding time) |
| | Start harvest in perfusion |
| Day 5 | Stop perfusion after 39 hours and empty the vector inside the bioreactor |

### TRANSFECTION STEP

Production of LVV vectors was performed using Polyethylenimine (PEI)-mediated transfection method. In Bio 1 the transfection was performed using multi-plasmid DNA in a total quantity of 45ng/cm² of the surface area, in Bio2, the transfection was performed using multi-plasmid DNA in a total quantity of 60ng/cm² of the surface area. In both Bio1 and Bio 2 the amount of PEI used has been set to maintain a ratio of 1:1 between PEI and DNA.

Protocol for transfection at day 3:
1. Prepare a total DNA mix for the bioreactors, diluting the correct amount of DNA in IMDM free
2. Dilute the correct amount of PEI in IMDM free
3. Transfer the diluted PEI in the tube containing the diluted DNA and mix.
4. Incubate the final mix for according to the manufacturer instructions.
5. Remove the correct volume from bioreactor, transfer the mix into the bioreactor.

The results obtained are shown in table 35, which shows physical particles and viral titer obtained in Bio1 and Bio2 and, in the third column, a medium the values of the same parameters obtained in the indicated number of runs ("n"), performed by using in transfection multi-plasmid DNA in a total quantity of 90 ng/cm2 of the surface area.

**Table 35: results of the process using minimum total DNA quantity in transfection**

| | **BIO 1 (45ng/cm²)** | **BIO 2 (60ng/cm²)** | **Medium of n runs 90ng/cm²** |
|---|---|---|---|
| **Physical Particles** | 51 | 56 | 55 |
| **P24 (ng/cm²)** | | | |
| **Viral Titer** | 1.7E+06 | 2.0E+06 | 3.0E+06 |
| **TU/cm2** | | | |

| | | | |
|---|---|---|---|
| (n*=42) (n*=36) * n is the number of runs performed | | | |

The results show that by reducing the total quantity of multi-plasmid DNA in transfection to 60ng/cm² and 45 ng/cm², productivity in terms of physical particles is substantially maintained. The viral titer observes a slight decline using 60 ng/cm² or 45 ng/cm² of total multi-plasmid DNA, but data obtained still reveal a good level of infective viral particles that, surprisingly, is comparable to that observed in example 2.3 for the runs PDE_B18197 Bio1 and PDE_B18197 Bio2, in which transfection was performed using 181 ng/cm² of the surface area and the TU/cm² observed was 2.2E+06 and 1.7E+06 respectively (as shown in table 23).

## Claims

1. A method for the manufacturing of lentiviral vectors in a packed bed bioreactor comprising the following steps:
a. inoculating the cells
b. culturing cells for expansion for at least one day
c. transfecting the cells using multi-plasmid DNA in a total amount between 45ng/cm² and 100ng/cm² of the surface area of the packed bed, and the PEI transfection reagent wherein the transfection is performed using a ratio between DNA amount and PEl amount corresponding to 1:1
d. harvesting the cell culture supernatant containing the recombinant lentiviral vectors.

2. A method according to claim 1 further comprising the change of the medium at the end of transfection wherein such change of medium is performed in perfusion mode or in batch mode.

3. A method according to claim 2 wherein the change of medium in batch mode is performed by draining from the bioreactor the cell culture medium containing the DNA transfecting particles and adding fresh medium to the cell culture.

4. A method according to any one of claims 1 to 3 wherein harvesting of the cell culture supernatant is performed in perfusion mode.

5. A method according to claim 3 comprising starting, immediately after the addition of the fresh medium, the collection of the recombinant lentiviral vectors by harvesting in perfusion mode the cell culture medium from the bioreactor.

6. A method according to any one of the preceding claims wherein the cells are transfected for a period up to 18 hours.

7. A method according to any one of claims 4 or 5 wherein harvesting in perfusion mode is performed for at least 39 hours after the post transfection change of medium.

8. A method according to any one of claims 4 or 5 wherein harvesting in perfusion mode is performed for 39 hours after the post transfection change of medium.

9. A method according to any one of the preceding claim further comprising a total change of medium between the step of culturing for expansion (b) and the step of transfection (c).

10. A method according to any one of claims 1 to 8 wherein the cells are cultivated for expansion in perfusion mode by supplying in the bioreactor up to the end of the expansion phase, fresh medium in a total amount of at least 0,1 ml/cm² of the surface area of the packed bed.

11. A method according to any one of claims 1 to 8 wherein the cells are cultivated for expansion in recirculation mode, using as reservoir, fresh medium in a total amount of at least 0,1 ml/cm² of the packed bed.

12. A method according to any one of the preceding claims wherein the transfection step (c) is performed in absence of perfusion or recirculation of fresh medium.

13. A method according to any one of the preceding claims wherein the cells are transfected under step (c) for a period of 8 hours.

14. A method according to any one of the preceding claims wherein the cells are selected from HEK293, HEK293 T, HEK 293E, HEK 293FT, 293 Vec, TE671, HT1080 or HeLa cell line.

15. A method according to any one of the preceding claims wherein the multi-plasmid DNA is composed by one plasmid carrying the lentiviral gag/pol genes, one plasmid carrying lentiviral rev gene, one plasmid carrying the gene encoding the envelope protein, and one plasmid carrying the transfer vector including the required portion of the lentiviral genome and the foreign gene of interest.

## Patentansprüche

1. Verfahren zur Herstellung von lentiviralen Vektoren in einem Festbett-Bioreaktor, das die folgenden Schritte umfasst:
a. Beimpfung der Zellen
b. Kultivierung von Zellen zur Expansion für mindestens einen Tag
c. Transfektion der Zellen unter Verwendung von Multi-Plasmid-DNA in einer Gesamtmenge zwischen 45ng/cm² und 100ng/cm² der Oberfläche des Festbettes und des PEI-Transfektionsreagenzes, wobei die Transfektion unter Verwendung eines Verhältnisses zwischen DNA-Menge und PEI-Menge entsprechend 1:1 durchgeführt wird
d. Ernte des Zellkulturüberstandes, der die rekombinanten lentiviralen Vektoren enthält.

2. Verfahren nach Anspruch 1, das ferner den Wechsel des Mediums am Ende der Transfektion umfasst, wobei dieser Wechsel des Mediums im Perfusionsmodus oder im Batch-Modus durchgeführt wird.

3. Verfahren nach Anspruch 2, wobei der Wechsel des Mediums im Batch-Modus durchgeführt wird, indem das Zellkulturmedium, das die DNAtransfizierenden Partikel enthält, aus dem Bioreaktor abgelassen und frisches Medium zu der Zellkultur gegeben wird.

4. Verfahren nach einem der Ansprüche 1 bis 3, wobei die Ernte des Zellkulturüberstands im Perfusionsmodus durchgeführt wird.

5. Verfahren nach Anspruch 3, bei dem unmittelbar nach der Zugabe des frischen Mediums mit der Sammlung der rekombinanten lentiviralen Vektoren begonnen wird, indem das Zellkulturmedium im Perfusionsmodus aus dem Bioreaktor geerntet wird.

6. Verfahren nach einem der vorhergehenden Ansprüche, wobei die Zellen für einen Zeitraum von bis zu 18 Stunden transfiziert werden.

7. Verfahren nach einem der Ansprüche 4 oder 5, wobei die Ernte im Perfusionsmodus für mindestens 39 Stunden nach dem Wechsel des Mediums nach der Nach-Transfektion durchgeführt wird.

8. Verfahren nach einem der Ansprüche 4 oder 5, wobei die Ernte im Perfusionsmodus 39 Stunden nach dem Wechsel des Mediums nach der Nach-Transfektion durchgeführt wird.

9. Verfahren nach einem der vorhergehenden Ansprüche, das ferner einen vollständigen Wechsel des Mediums zwischen dem Schritt der Kultivierung zur Expansion (b) und dem Schritt der Transfektion (c) umfasst.

10. Verfahren nach einem der Ansprüche 1 bis 8, wobei die Zellen zur Expansion im Perfusionsmodus kultiviert werden, indem dem Bioreaktor bis zum Ende der Expansionsphase frisches Medium in einer Gesamtmenge von mindestens 0,1 ml/cm² der Oberfläche des Festbettes zugeführt wird.

11. Verfahren nach einem der Ansprüche 1 bis 8, wobei die Zellen zur Expansion im Rezirkulationsmodus kultiviert werden, wobei als Reservoir frisches Medium in einer Gesamtmenge von mindestens 0,1 ml/cm² des Festbettes verwendet wird.

12. Verfahren nach einem der vorhergehenden Ansprüche, wobei der Transfektionsschritt (c) ohne Perfusion oder Rezirkulation von frischem Medium durchgeführt wird.

13. Verfahren nach einem der vorhergehenden Ansprüche, wobei die Zellen in Schritt (c) für einen Zeitraum von 8 Stunden transfiziert werden.

14. Verfahren nach einem der vorhergehenden Ansprüche, wobei die Zellen aus HEK293, HEK293 T, HEK 293E, HEK 293FT, 293 Vec, TE671, HT1080 oder HeLa-Zelllinie ausgewählt werden.

15. Verfahren nach einem der vorhergehenden Ansprüche, wobei die Multiplasmid-DNA aus einem Plasmid, das die lentiviralen gag/pol-Gene trägt, einem Plasmid, das das lentivirale rev-Gen trägt, einem Plasmid, das das Gen trägt, das für das Hüllprotein kodiert, und einem Plasmid, das den Transfervektor trägt, der den erforderlichen Teil des lentiviralen Genoms und das Fremdgen von Interesse enthält, zusammengesetzt ist.

## Revendications

1. Procédé de fabrication de vecteurs lentiviraux dans un bioréacteur à lit fixe comprenant les étapes suivantes :
a. l'inoculation des cellules
b. la mise en culture des cellules en vue de leur expansion pendant au moins un jour
c. la transfection des cellules à l'aide d'ADN multiplasmidique dans une quantité totale comprise entre 45ng/cm² et 100ng/cm² de la surface du lit fixe, et du réactif de transfection PEI, la transfection étant réalisée en utilisant un rapport entre la quantité d'ADN et la quantité de PEI correspondant à 1:1
d. la récolte du surnageant de culture cellulaire contenant les vecteurs lentiviraux recombinants.

2. Procédé selon la revendication 1, comprenant en outre le changement du milieu à la fin de la transfection, ce changement de milieu étant effectué en mode perfusion ou en mode batch.

3. Procédé selon la revendication 2, dans lequel le changement de milieu en mode batch est effectué en drainant du bioréacteur le milieu de culture cellulaire contenant les particules de transfection de l'ADN et en ajoutant du milieu frais à la culture cellulaire.

4. Procédé selon l'une quelconque des revendications 1 à 3, dans lequel la récolte du surnageant de culture cellulaire est effectuée en mode perfusion.

5. Procédé selon la revendication 3, comprenant le démarrage, immédiatement après l'ajout du milieu frais, de la collecte des vecteurs lentiviraux recombinants par la récolte en mode perfusion du milieu de culture cellulaire du bioréacteur.

6. Procédé selon l'une quelconque des revendications précédentes, dans lequel les cellules sont transfectées pendant une période allant jusqu'à 18 heures.

7. Procédé selon l'une quelconque des revendications 4 ou 5, dans lequel la récolte en mode perfusion est effectuée pendant au moins 39 heures après le changement de milieu après la transfection.

8. Procédé selon l'une quelconque des revendications 4 ou 5, dans lequel la récolte en mode perfusion est effectuée pendant 39 heures après le changement de milieu après la transfection.

9. Procédé selon l'une quelconque des revendications précédentes, comprenant en outre un changement total de milieu entre l'étape de culture pour l'expansion (b) et l'étape de transfection (c).

10. Procédé selon l'une quelconque des revendications 1 à 8, dans lequel les cellules sont cultivées pour l'expansion en mode perfusion par l'apport dans le bioréacteur, jusqu'à la fin de la phase d'expansion, de milieu frais dans une quantité totale d'au moins 0,1 ml/cm² de la surface du lit fixe.

11. Procédé selon l'une quelconque des revendications 1 à 8, dans lequel les cellules sont cultivées pour expansion en mode de recirculation, en utilisant comme réservoir un milieu frais dans une quantité totale d'au moins 0,1 ml/cm² du lit fixe.

12. Procédé selon l'une quelconque des revendications précédentes, dans lequel l'étape de transfection (c) est réalisée en l'absence de perfusion ou de recirculation de milieu frais.

13. Procédé selon l'une quelconque des revendications précédentes, dans lequel les cellules sont transfectées à l'étape (c) pendant une période de 8 heures.

14. Procédé selon l'une quelconque des revendications précédentes, dans lequel les cellules sont choisies parmi les lignées cellulaires HEK293, HEK293 T, HEK 293E, HEK 293FT, 293 Vec, TE671, HT1080 ou HeLa.

15. Procédé selon l'une quelconque des revendications précédentes, dans lequel l'ADN multiplasmidique est composé d'un plasmide portant les gènes lentiviraux gag/pol, d'un plasmide portant le gène lentiviral rev, d'un plasmide portant le gène codant pour la protéine d'enveloppe et d'un plasmide portant le vecteur de transfert comprenant la partie requise du génome lentiviral et le gène étranger d'intérêt.
